# EUROPÄISCHE PATENTSCHRIFT

(11) **EP 2 247 323 B1**
(45) Veröffentlichungstag und Bekanntmachung des Hinweises auf die Patenterteilung: **24.08.2011**
(21) Anmeldenummer: 09714488.5
(22) Anmeldetag: 26.02.2009
(51) Int. Cl.: A61M 1/28, G05B 19/042

(54) **VERFAHREN ZUR ANSTEUERUNG VON VENTILEN ZUR FLUSSWEGSTEUERUNG UND MASCHINEN, INSBESONDERE MEDIZINISCHE BEHANDLUNGSMASCHINEN**
METHOD FOR ACTUATING VALVES FOR CONTROLLING A FLOW PATH AND MACHINES, ESPECIALLY MEDICAL TREATMENT MACHINES
PROCÉDÉ DE COMMANDE DE VALVES POUR LA COMMANDE DE VOIES D'ÉCOULEMENT ET INSTRUMENTS, NOTAMMENT INSTRUMENTS DE TRAITEMENT MÉDICAL

(30) Priorität: 29.02.2008 DE 102008011827
(43) Veröffentlichungstag der Anmeldung: 10.11.2010
(73) Patentinhaber: Fresenius Medical Care Deutschland GmbH, 61352 Bad Homburg (DE)
(72) Erfinder: HEDMANN, Frank L., 97332 Volkach (DE); KLATTE, Stephan, 31582 Nienburg (Weser) (DE)
(74) Vertreter: Laufhütte, Dieter
(86) Internationale Anmeldenummer: PCT/EP2009/001381
(87) Internationale Veröffentlichungsnummer: WO 2009/106329

(56) Entgegenhaltungen:
- US-A- 5 450 346
- US-A1- 2003 220 607
- US-A1- 2007 106 401
- US-A1- 2007 112 297

## Beschreibung

Die vorliegende Erfindung betrifft ein Verfahren zur Ansteuerung von Ventilen zur Flusswegsteuerung insbesondere in einer medizinischen Behandlungsmaschine. Weiterhin betrifft die vorliegende Erfindung ein Verfahren zur Überwachung des aktuellen und/oder geplanten Zustands einer Mehrzahl von Ventilen bei der Flusswegsteuerung insbesondere in einer medizinischen Behandlungsmaschine, sowie eine Maschine, insbesondere eine medizinische Behandlungsmaschine, mit einer Steuerung zur Durchführung der entsprechenden Verfahren.

Insbesondere betrifft die vorliegende Erfindung dabei Verfahren zum Betrieb einer Maschine, bei welcher ein Kassettensystem zum Transport von insbesondere medizinischen Flüssigkeiten eingesetzt wird. Die vorliegende Erfindung kommt dabei besonders vorteilhaft bei Behandlungsmaschinen im Bereich der Dialyse, insbesondere im Bereich der Peritonealdialyse, zum Einsatz. Bei solchen Behandlungsmaschinen kommen üblicherweise Kassettensysteme zum Transport der Behandlungsflüssigkeiten bzw. zur Durchführung der Behandlung zum Einsatz. Insbesondere betrifft die vorliegende Erfindung dabei Peritonealdialysemaschinen, wie sie in US 2007/0112297 A1 und US 2006/0195064 A1 dargestellt sind, sowie Verfahren zum Betrieb solcher Peritonealdialysemaschinen.

Die in solchen Kassettensystemen verwendeten Einmalkassetten weisen dabei Fluidwege und Ventilstellen auf, über welche die für den jeweiligen Behandlungsschritt benötigten Flusswege in der Kassette hergestellt werden können. Die Fluidwege der Kassetten werden dabei üblicherweise durch flüssigkeitsführende Kanäle gebildet, welche im Bereich der Ventilstellen mindestens eine flexible Wand aufweisen. Die flexible Wand kann dann durch einen Ventilaktor in den flüssigkeitsführenden Kanal gedrückt werden und diesen so versperren. Als Aktoren können dabei z. B. pneumatisch betätigbare Aktoren zum Einsatz kommen, welche sich durch Beaufschlagen des Aktors mit Druck ausdehnen und so als Ventilstößel dienen. Ebenso können hydraulische oder elektromotorische Ventilaktoren eingesetzt werden. Die flüssigkeitsführenden Kanäle mit den Ventilstellen sind dabei üblicherweise an der Einweg-Kassette angeordnet, die Ventilaktoren an der Behandlungsmaschine. Die Kassetten werden dann in die Behandlungsmaschine eingelegt und mit dieser verkoppelt, so dass die Ventilaktoren an der Behandlungsmaschine mit den Ventilstellen an der Kassette in Eingriff kommen und den Schaltzustand der Ventile der Kassette bestimmen.

Zur Ansteuerung der Ventile sind dabei in der Maschinensteuerung üblicherweise Programme hinterlegt, über welche die zur Herstellung eines benötigten Flussweges (z. B. von einer Pumpkammer zum Patienten) benötigte Gruppe von Ventilen angesteuert wird. Unter einem Flussweg versteht man dabei die Verbindung zwischen zwei Quellen und/oder Senken, welche durch Ventile verbunden sind, z. B. die Verbindung zwischen einem Beutel und einer Pumpe, welche durch Öffnen der zwischen Beutel und Pumpe liegenden Ventile sowie Schließen von Ventilen zu anderen Komponenten führenden Verbindungsleitungen hergestellt wird. Üblicherweise sind dabei eine Mehrzahl von Ventilen vorgesehen, bei bekannten Systemen z. B. neun oder sechzehn Ventile, über deren unterschiedliche Ansteuerung eine Vielzahl von unterschiedlichen Flusswegen zwischen dem Patienten, dem Pumpkammern der Kassette sowie unterschiedlichen Beuteln mit Flüssigkeit oder zur Drainage hergestellt werden kann.

Bei bekannten Verfahren wird ein Flussweg, der z.B. für einen bestimmten Pumpzyklus benötigt wird, durch das verwendete Verfahren fest vorgegeben. Hierbei kennt die Maschine für das verwendete Verfahren den entsprechenden Flußweg, da dieser fest in der Anwendung hinterlegt ist. Verlangt das Verfahren nun eine Verbindung von einem Punkt A zu einem Punkt D, welche theoretisch sowohl über B als auch über C erfolgen könnte, so ist diese Verbindung vorher in der Anwendung fest hinterlegt - z. B. über B. Die Anwendung hat also nicht die freie Wahl, sondern erhält im vorliegenden Fall eine fest hinterlegte Verbindung zugewiesen.

Eine Kontrolle des erlaubten Flußweges A - B - D ist insofern nicht notwendig, da im Schutzsystem nur erlaubte und vorher festgelegte Verbindungen hinterlegt sind. So werden bei der Programmierung der Verfahren die für eine komplette Anwendung benötigten Flußwege hinterlegt. Soll nun ein neues Verfahren in die Anwendung der Maschine integriert werden, so müssen die hierfür benötigten Flußwege dahingehend überprüft werden, dass sie entweder in dem Schutzsystem hinterlegt sind oder sie müssen neu hinterlegt werden, was zu einer Gesamtüberprüfung führen muss, da eventuell auch die Wechsel zwischen einzelnen zu schaltenden Flußwegen auf Sinnhaftigkeit überprüft werden müssen. Ein weiterer Nachteil der bisher bekannten Systeme ist weiterhin der Wechsel zwischen zwei vorgegebenen Pumpzyklen, da hierzu aus Sicherheitsgründen alle Ventile zuerst geschlossen werden. Dies führt zu einer Erhöhung des Verschleißes bei den Ventilkomponenten. Die bekannten Systeme sind daher unflexibel und erschweren die Implementierung neuer Verfahren.

Aufgabe der vorliegenden Erfindung ist es, Verfahren zur Ansteuerung bzw. Überwachung von Ventilen zur Flußwegsteuerung zur Verfügung zu stellen, bei welchen sich die Implementierung von neuen Verfahren flexibler gestaltet, wobei die notwendigen Sicherheitsaspekte dennoch berücksichtigt werden. Weiterhin ist es Aufgabe der vorliegenden Erfindung, Maschinen, insbesondere medizinische Behandlungsmaschinen, mit entsprechenden Ventil- bzw. Ventilaktorsteuerungen zur Verfügung zu stellen.

Diese Aufgabe wird von Verfahren gemäß den Ansprüchen 1 und 24 und Maschinen gemäß den Ansprüchen 12 und 31 gelöst. Vorteilhafte Ausgestaltungen der erfindungsgemäßen Verfahren ergeben sich dabei aus den Unteransprüchen.

Die vorliegende Erfindung umfasst dabei ein Verfahren zur Ansteuerung von Ventilen zur Flusswegsteuerung insbesondere in einer medizinischen Behandlungsmaschine, mit einer Mehrzahl von Prozessen zur Herstellung von Flusswegen durch Ansteuerung einer Gruppe von Ventilen, wobei jeder Prozess eine zu seiner Durchführung geeignete Gruppe von Ventilen für sich beansprucht, so dass andere Prozesse den Schaltzustand dieser Ventile nicht ändern können, und wobei jeder Prozess selbst über die Freigabe der durch ihn beanspruchten Ventile entscheidet. Durch dieses Verfahren ist es einerseits möglich, Ventile oder Gruppen von Ventilen einzelnen Prozessen flexibel zuzuordnen.

Ein Prozeß stellt dabei einen Verfahrensbestandteil innerhalb der Ventilsteuerung der Maschine dar, welcher einen gewissen Flussweg bereitstellt. Hierzu benötigt ein Prozeß eine Gruppe von Ventilen, deren Schaltzustand er so einstellt, dass die Ventile den Flussweg herstellen. Diese Gruppe an Ventilen stellt dabei üblicherweise nur einen Teil aller Ventile dar, so dass ein Prozeß zu seiner Durchführung üblicherweise nicht alle Ventile beansprucht. Dabei kann es für den gleichen Flussweg, z. B. von einer Pumpkammer zu einem Beutel, mehrere unterschiedliche Möglichkeiten geben, diesen zu realisieren, d.h. ein Prozeß kann durch unterschiedliche Gruppen von Ventilen realisiert werden.

Durch das erfindungsgemäße Verfahren ist es nun möglich, dass einzelne Prozessen flexibel unterschiedliche Gruppen von Ventilen beanspruchen, ohne dass die Sicherheit des Systems beeinträchtigt würde. Während ein Prozeß die zur Herstellung des Flusswegs benötigte Gruppe von Ventilen beansprucht, können andere Prozesse den Schaltzustand dieser Ventile nicht ändern, so dass allein der einen Flussweg bereit stellende Prozeß auch über dessen Auflösung entscheidet.

Dabei entscheidet jeder Prozeß nach internen und/oder externen Prozesskriterien über die Freigabe der durch ihn beanspruchten Ventile. Interne Prozesskriterien können z. B. Pumpenpositionen, bereits durchgeführte Schritte oder der Abschluß des Prozesses sein. Äußere Umstände können z. B. eine höhere Priorisierung eines anderen Prozesses sein. Entscheidend ist bei dem vorliegenden Verfahren jedoch, dass nur der jeweils die Ventile beanspruchende Prozeß diese auch wieder freigeben kann, so dass ein koordiniertes und sicheres Zusammenspiel mehrerer Prozesse gewährleistet ist.

Vorteilhafterweise gibt dabei erfindungsgemäß jeder Prozeß nach seiner Beendigung die beanspruchte Gruppe von Ventilen frei. Hierdurch können diese von anderen Prozessen wieder ihrerseits beansprucht und zu ihrer Durchführung genutzt werden.

Vorteilhafterweise kann dabei ein Prozeß je nach Verfügbarkeit der Ventile unterschiedliche Gruppen von Ventilen beanspruchen. Dies ermöglicht ein flexibles Ventilmanagement, bei welchem die verfügbaren Ressourcen optimal genutzt werden. Ein Ventil wird dabei in einer ersten Verfahrensvariante für einen Prozeß als verfügbar betrachtet, wenn es von keinem anderen Prozeß beansprucht wird. Damit kann ein Ventil jedoch immer nur von einem einzigen Prozeß beansprucht werden, auch wenn zwei Prozesse für ein Ventil eigentlich den gleichen Schaltzustand benötigen und daher prinzipiell kompatibel sind.

In einer zweiten Verfahrensvariante wird ein Ventil daher für einen zweiten Prozeß als verfügbar betrachtet, wenn es von keinem anderen Prozeß beansprucht wird, oder wenn es zwar von einem ersten Prozess bereits beansprucht wird, aber den vom zweiten Prozeß benötigten Schaltzustand aufweist. Ein solches bereits von einem ersten Prozeß beanspruchtes Ventil kann dabei vom zweiten Prozeß nicht mehr in seinem Schaltzustand geändert werden. Ist dies jedoch zur Herstellung des Flussweges durch den zweiten Prozess nicht nötig, so kann der zweite Prozeß dieses Ventil nutzen. Hierfür beansprucht vorteilhafterweise auch der zweite Prozeß das Ventil, so dass dessen Schaltzustand auch vom ersten Prozeß nicht mehr geändert werden kann. Damit können beide Prozesse das gleiche Ventil nutzen, wobei durch die Beanspruchung des Ventils durch beide Prozesse eine unbeabsichtigte Auflösung des Flusswegs des einen Prozesses durch den anderen Prozeß verhindert wird.

Vorteilhafterweise stellen dabei die einzelnen Prozesse Verfahrensbestandteile innerhalb der Ventilsteuerung der Maschine dar, welche einen gewissen Flussweg herstellen und bis zu ihrer Beendigung beibehalten. Die Änderung eines Flussweges erfolgt damit immer durch die Durchführung eines neuen Prozesses.

Vorteilhafterweise fragt dabei erfindungsgemäß jeder Prozess vor seiner Durchführung die Verfügbarkeit einer zu seiner Durchführung geeigneten Gruppe an Ventilen ab. Ein Prozeß, welcher einen gewissen Flussweg herstellen will, fragt damit zunächst ab, ob eine hierfür geeignete Gruppe von Ventilen bereits verfügbar ist.

Dabei wird vorteilhafterweise ein Prozess durchgeführt, wenn eine geeignete Gruppe an Ventilen verfügbar ist, wobei der Prozeß diese Gruppe an Ventilen für sich beansprucht. Ist also eine solche Gruppe von Ventilen verfügbar, so kann der Prozeß unabhängig von weiteren möglicherweise parallel laufenden Prozessen durchgeführt werden. Hierfür ist es ausreichend, wenn aus mehreren möglichen Kombinationen von Ventilen, welche zur Herstellung des gewünschten Flusswegs geeignet sind, eine Kombination von Ventilen verfügbar ist. Kann der gewünschte Flussweg mit den verfügbaren Ventilen jedoch nicht hergestellt werden, so kann der Prozeß zunächst nicht starten.

Vorteilhafterweise führt dabei die Abfrage eines nicht verfügbaren Ventils durch einen zweiten Prozess zu einer Aufforderung an den ersten Prozeß, welcher dieses Ventil beansprucht hat, dieses Ventil freizugeben, wobei nur der erste Prozess über diese Aufforderung entscheidet. Insbesondere ist dies dann der Fall, wenn keine geeignete Gruppe von Ventilen zur Durchführung des zweiten Prozeß zur Verfügung steht. Der zweite Prozeß fordert dann den ersten Prozeß auf, das von ihm benötigte Ventil freizugeben. Der erste Prozeß entscheidet nun selbständig über diese Aufforderung, wobei interne und externe Prozesskriterien berücksichtigt werden können. Erst wenn der erste Prozeß das benötigte Ventil oder die benötigte Gruppe von Ventilen freigegeben hat, kann der zweite Prozeß diese in ihrem Schaltzustand ändern und den gewünschten Fluidweg herstellen.

Vorteilhafterweise beansprucht dabei der abfragende Prozeß mit der Abfrage alle abgefragten und verfügbaren Ventile für sich. Hierdurch ist bei einer Mehrzahl von Prozessen sichergestellt, dass ein Prozeß zunächst alle verfügbaren Ventile für sich beanspruchen kann und somit nur noch auf die Ventile warten muss, welche von anderen Prozessen freigegeben werden müssen, so dass er deren Schaltzustand ändern kann.

Vorteilhafterweise erhält weiterhin der abfragende Prozeß mit der Abfrage für alle abgefragten aber nicht verfügbaren Ventile ein Beanspruchungsrecht, so dass er diese Ventile für sich beanspruchen kann, wenn diese freigegeben wurden. Hierdurch ist auch bei mehreren Prozessen ein sicherer Übergang von einem Prozeß zum nächsten möglich. Insbesondere können hierbei für unterschiedliche Prozesse unterschiedliche Prioritätsrechte vergeben werden.

Vorteilhafterweise umfasst das erfindungsgemäße Verfahren dabei einen zentralen Ventilzuordnungsprozess, bei welchem alle anderen Prozesse die benötigten Ventile beanspruchen, freigeben und/oder anfragen. Dieser zentrale Ventilzuordnungsprozess übernimmt damit das Verteilungsmanagement für die einzelnen Ventile und Ventilgruppen, wobei jedoch die Freigabe einzelner Ventile nur durch den Prozeß erfolgen kann, welcher diese aktuell nutzt.

Das erfindungsgemäße Verfahren umfasst dabei vorteilhafterweise folgende Schritte: Beanspruchen einer ersten Gruppe von Ventilen zur Durchführung eines ersten

Prozesses, bei welchem ein erster Flussweg durch Ansteuerung der zugehörigen ersten Gruppe an Ventilen hergestellt wird; Abfrage der Verfügbarkeit einer zweiten Gruppe an Ventilen zur Durchführung eines zweiten Prozesses, bei welchem ein zweiter Flussweg durch Ansteuerung der zugehörigen zweiten Gruppe an Ventilen hergestellt werden soll; und Durchführen des zweiten Prozesses, wenn alle benötigten Ventile verfügbar sind, wobei die Abfrage durch den zweiten Prozeß von Ventilen, welche nicht verfügbar sind, zu einer Aufforderung an den ersten Prozeß führen, diese Ventile freizugeben, wobei nur der erste Prozeß über diese Anfrage entscheidet.

Die vorliegende Erfindung ermöglicht damit eine flexible Flusswegplanung. Unter einem Flussweg versteht man dabei die Verbindung zwischen zwei Quellen und/oder Senken, welche durch Ventile verbunden sind, z. B. die Verbindung zwischen einem Beutel und einer Pumpe. Wird ein solcher Flussweg von einem ersten Prozeß bereitgestellt, so werden die hierfür angesteuerten Ventile von diesem ersten Prozeß beansprucht und können nicht mehr von weiteren Prozessen zur Herstellung anderer Flusswege in ihrem Schaltzustand geändert werden. Wird nun von einem zweiten Prozeß, z. B. einem zweiten Pumpzyklus des Verfahrens, ein bestimmter Flussweg angefordert, so werden alle für den Flussweg notwendigen Ventile abgefragt. Die verfügbaren Ventile können beansprucht werden. Die bereits an den ersten Prozeß, z. B. einen ersten Pumpzyklus vergebenen Ventile, welche in ihrem Schaltzustand geändert werden müßten, werden bei diesem ersten Prozeß angefragt. Der erste Prozeß hat dabei die alleinige Entscheidungsgewalt, wann und ob er dem zweiten Prozeß die Ventile freigibt. Sobald die entsprechenden Ventile durch den ersten Prozeß freigegeben worden sind, kann der anfragende zweite Prozeß die Ventile verwenden und seinen Flußweg bilden.

Um einen ungewollten freien Fluß bei einem Wechsel von einem ersten Prozeß auf einen zweiten Prozeß zu verhindern, werden die benötigten Ventile, die sich bereits im richtigen Zustand für den entsprechenden Prozeß befinden, in ihrem Zustand belassen. Für die Ventile, die ihren Zustand ändern müssen gilt, dass in einem ersten Schritt die zu schließenden Ventile geschlossen werden. In einem zweiten Schritt werden die von dem zweiten Prozeß in einem offenen Zustand benötigten Ventile geöffnet.

Durch dieses erfindungsgemäße Verfahren zur Ansteuerung der Ventile ist es möglich, dass eine Mehrzahl von Prozessen flexibel auf die einzelnen Ventile zugreift, wobei dennoch durch die erfindungsgemäße Freigaberegeln ein sicherer Betrieb gewährleistet ist. Diese flexible und offene Architektur der erfindungsgemäßen Verfahren ermöglicht es dabei, besonders einfach neue Verfahren zu implementieren. Insbesondere muß hierzu nicht mehr die komplette Systemarchitektur überarbeitet werden. Vielmehr können neue Prozesse problemlos zu den bestehenden Prozessen hinzugefügt werden.

In der flexiblen Gestaltung der Implementierung von neuen Verfahren, die auf der Behandlungsmaschine wie z. B. einem Cycler ablaufen können, gibt es durch diese Art der Flußwegbildung zudem wesentlich weniger Ventilschaltvorgänge und damit einen geringeren Verschleiß der Ventilkomponenten. Ein solcher Cycler stellt dabei eine Behandlungsmaschine für die Dialyse, insbesondere für die Peritonealdialyse dar, welche einen automatisierten Austausch der Dialyseflüssigkeiten erlaubt. Insbesondere kann darauf verzichtet werden, bei einem Wechsel zwischen Pumpzyklen zunächst alle Ventile zu schließen, wie dies im Stand der Technik aus Sicherheitsgründen noch notwendig war.

Die vorliegende Erfindung umfasst dabei weiterhin eine Maschine, insbesondere eine medizinische Behandlungsmaschine, mit einer Mehrzahl von Ventilaktoren zur Ansteuerung von Ventilen zur Flußwegsteuerung insbesondere in einem Kassettensystem, mit einer Ventilaktorsteuerung zur Durchführung einer Mehrzahl von Prozessen zur Herstellung von Flußwegen durch Ansteuerung einer Gruppe von Ventilaktoren, wobei die Ventilaktorsteuerung so ausgeführt ist, dass jeder Prozeß eine zu seiner Durchführung geeignete Gruppe von Ventilaktoren für sich beansprucht, so dass andere Prozesse den Schaltzustand dieser Ventilaktoren nicht ändern können, und jeder Prozeß selbst über die Freigabe der durch ihn beanspruchten Ventilaktoren entscheidt. Über die Ventilaktoren werden dabei Ventile, welche insbesondere in einem Kassettensystem angeordnet sein können, das in die Maschine eingelegt wird, angesteuert, um entsprechende Flusswege in der Kassette bereit zu stellen. Durch eine solche Maschine ergeben sich die gleichen Vorteile, wie sie bereits bezüglich des Verfahrens beschrieben wurden. Insbesondere ist es hierdurch möglich, den einzelnen Prozessen unterschiedliche Gruppen von Ventilaktoren flexibel zuzuordnen, ohne dass die Sicherheit des Systems beeinträchtigt würde. Insbesondere ist die erfindungsgemäße Ventilaktorsteuerung dabei so programmiert, dass die erfindungsgemäße Prozesse erfindungsgemäß durchgeführt werden, wobei das erfindungsgemäße Verfahren vorteilhafterweise automatisch auf der erfindungsgemäßen Maschine abläuft.

Vorteilhafterweise entscheidet dabei erfindungsgemäß jeder Prozess nach internen oder externen Prozeßkriterien über die Freigabe der durch ihn beanspruchten Ventilaktoren. Hierdurch wird wie bereits bezüglich des Verfahrens beschrieben die Sicherheit des Betriebs der Maschine sichergestellt.

Vorteilhafterweise gibt dabei jeder Prozeß nach seiner Beendigung die beanspruchte Gruppe von Ventilaktoren frei. Dadurch können diese wieder von anderen Prozessen in ihrem Schaltzustand geändert werden.

Vorteilhafterweise kann dabei ein Prozeß je nach Verfügbarkeit der Ventilaktoren unterschiedliche Gruppen von Ventilaktoren beanspruchen. Hierdurch ist eine flexible Zuordnung von unterschiedlichen Gruppen von Ventilaktoren zu einem Prozeß möglich, so dass durch den gleichen Prozeß zur Herstellung eines Flußwegs unterschiedliche Gruppen von Ventilaktoren angesteuert werden können.

Vorteilhafterweise fragt dabei jeder Prozeß vor seiner Durchführung die Verfügbarkeit einer zu seiner Durchführung geeigneten Gruppe an Ventilaktoren ab. Dabei wird ein Prozeß durchgeführt, wenn eine geeignete Gruppe an Ventilaktoren verfügbar ist, wobei der Prozeß diese Gruppe an Ventilaktoren für sich beansprucht. Hierdurch ist sichergestellt, dass andere Prozesse den Schaltzustand dieser Gruppe von Venilaktoren nicht mehr ändern kann, so dass nur der Prozeß selbst darüber entscheiden kann, ob der von ihm zur Verfügung gestellte Flußweg beibehalten wird oder aufgelöst wird.

Vorteilhafterweise führt dabei die Abfrage eines nicht verfügbaren Ventilaktors durch einen zweiten Prozeß zu einer Aufforderung an den ersten Prozeß, welcher diesen Ventilaktor beansprucht hat, diesen Ventilaktor freizugeben, wobei nur der erste Prozeß über diese Aufforderung entscheidet. Insbesondere führt eine Abfrage dann zu einer Aufforderung, ein Ventilaktor freizugeben, wenn keine Gruppe an Ventilaktoren verfügbar ist, mit welcher der zweite Prozess den beabsichtigten Flußweg herstellen könnte. In diesem Fall muß erst der vom ersten Prozeß bereit gestellte Flussweg aufgelöst werden, um den vom zweiten Prozeß bereit zu stellenden Flussweg bilden zu können.

Vorteilhafterweise beansprucht dabei der abfragende Prozeß mit der Abfrage alle abgefragten und verfügbaren Ventilaktoren für sich. Hierdurch können die verfügbaren Ventilaktoren, das heißt alle von keinem anderen Prozeß beanspruchten und vorteilhafterweise alle von anderen Prozessen beanspruchten, aber sich bereits im gewünschten Schaltzustand befindlichen Ventilaktoren, von dem abfragenden Prozeß für sich beansprucht werden, so dass der Schaltzustand dieser Ventilaktoren von anderen Prozessen nicht mehr geändert werden kann.

Weiterhin vorteilhafterweise erhält der abfragende Prozeß mit der Abfrage für alle abgefragten aber nicht verfügbaren Ventilaktoren ein Beanspruchungsrecht, so dass er diese Ventilaktoren für sich beanspruchen kann, wenn diese freigegeben wurden. Muss also der Schaltzustand eines bereits von einem anderen Prozeß beanspruchten Ventilaktors geändert werden, so beansprucht der abfragende Prozeß dieses Ventil nicht für sich, sondern erhält hierfür lediglich ein Beanspruchungsrecht, welches er ausüben kann, wenn dieser Ventlaktor von dem ersten Prozeß freigegeben wurde. Hierdurch ist eine Synchronisation einer Vielzahl von unterschiedlichen Prozessen möglich.

Vorteilhafterweise umfasst die Ventilaktorsteuerung dabei einen zentralen Ventilzuordnungsprozeß, bei welchem alle anderen Prozesse die benötigten Ventilaktoren beanspruchen, freigeben und/oder anfragen können.

Vorteilhafterweise ist die erfindungsgemäße Ventilaktorsteuerung dabei so programmiert, dass folgende Schritte durchgeführt werden: Beanspruchen einer ersten Gruppe von Ventilaktoren zur Durchführung eines ersten Prozesses, bei welchem ein erster Flußweg durch Ansteuerung der zugehörigen ersten Gruppe an Ventilen hergestellt wird; Abfrage der Verfügbarkeit einer zweiten Gruppe an Ventilaktoren zur Durchführung eines zweiten Prozesses, bei welchem ein zweiter Flußweg durch Ansteuerung der zugehörigen zweiten Gruppe an Ventilen hergestellt werden soll; und Durchführen des zweiten Prozesses, wenn alle benötigten Ventilaktoren verfügbar sind, wobei die Abfrage durch den zweiten Prozeß von Ventilaktoren, welche nicht verfügbar sind, zu einer Aufforderung an den ersten Prozess führt, diese Ventilaktoren freizugeben, wobei nur der erst Prozess über diese Anfrage entscheidet.

Die erfindungsgemäße Maschine weist dabei vorteilhafterweise eine Ankoppelfläche zum Ankoppeln einer Kassette auf, wobei die Kassette ventil- und flüssigkeitsführende Kanäle umfasst, und wobei die maschinenseitigen Ventilaktoren zur Herstellung unterschiedlicher Flußwege in der Kassette den Schaltzustand der kassettenseitigen Ventile bestimmen. Vorteilhafterweise sind dabei die Ventilaktoren auf der Ankoppelfläche der Maschine angeordnet. Die Kassette stellt dabei üblicherweise ein Einwegteil dar, welches in die Maschine eingelegt wird.

Erfindungsgemäß ergibt sich damit ein Verfahren sowie eine entsprechende Maschine, auf welcher die Flußwegbildung erheblich flexibler implementierbar ist.

Auch das Sicherheitssystem sollte an die erfindungsgemäße flexible Flusswegplanung angepaßt werden, insbesondere um die in der Flusswegplanung gewonnene Flexibilität nicht wieder zu verlieren.

Die vorliegende Erfindung umfasst daher weiterhin ein Verfahren zur Überwachung des aktuellen und/oder geplanten Zustands einer Mehrzahl von Ventilen bei der Flusswegsteuerung, insbesondere in einer medizinischen Behandlungsmaschine, mit den Schritten: Ermitteln des aktuellen und/oder geplanten Schaltzustands der Ventile, Ermitteln der durch den Schaltzustand der Ventile sich ergebenden Verbindungen, und Vergleichen der sich ergebenden Verbindungen mit einer vorgegebenen Menge von unzulässigen Verbindungen.

Es werden also nicht mehr alle erlaubten Flußwege hinterlegt, die dann bei Änderung oder Neueinführung eines Verfahrens überarbeitet werden müßten, sondern es werden für die Gesamtanordnung der Ventile, Pumpen und Anschlüsse, das heißt für alle Ventile, Senken und/der Quellen des Systems, einmalig alle verbotenen Wege ermittelt und hinterlegt. Zur Überwachung des aktuellen und/oder geplanten Zustands der Ventile bzw. des Systems müssen damit nur noch die sich aus dem Schaltzustand der Ventile ergebenden Verbindungen ermittelt und mit dieser vorgegebenen Menge von unzulässigen Verbindungen verglichen werden.

Als unzulässige Verbindungen müssen dabei nur Verbindungen zwischen den Ein- bzw. Ausgängen des Systems berücksichtigt werden, z.B. eine Verbindung zwischen dem zum Patienten führenden Anschluß und dem zum Drainagebeutel führenden Anschluß. Der interne Schaltzustand der Ventile, welcher einer solchen Verbindungen entspricht, muß dagegen nicht mehr als zulässig oder unzulässig klassifiziert werden, da das Verfahren ermittelt, welche Verbindungen zwischen den Ein- bzw. Ausgängen des Systems durch den Schaltzustand der Ventile (und die damit verbundenen internen Verbindungen) vorliegen. Nur diese Verbindungen zwischen den Ein- bzw. Ausgängen des Systems müssen dann mit den unzulässigen Verbindungen verglichen werden.

Vorteilhafterweise erfolgt die Ermittlung der sich durch einen Schaltzustand ergebenden Verbindungen immer dann, wenn sich der Schaltzustand eines Ventils ändert oder ändern soll. Es werden also die Ventilstati überwacht. Ändert sich ein Ventilstatus, oder will ein Prozeß einen Ventilstatus ändern, so werden die sich aus dem geänderten Schaltzustand des Ventils ergebenden Verbindungen bzw. Flusswege ermittelt und es wird überprüft, ob es sich bei diesen Verbindungen bzw. Flusswegen um unzulässige Verbindungen bzw. Flusswege handelt.

Vorteilhafterweise werden dabei zur Ermittlung der sich durch einen Schaltzustand ergebenden Verbindungen alle offenen Ventile von einem oder mehreren Startpunkten aus virtuell geflutet. Hierbei wird von einem Startpunkt aus überprüft, welche Verbindungen sich von diesem Startpunkt über die offenen Ventile des Systems ergeben. Dabei sind die Ventile sowie die sich durch die Flüssigkeitsführenden Kanäle zwischen den Ventilen ergebenden Verbindungen in der Behandlungsmaschine z.B. in Form eines Netzwerks abgelegt.

Vorteilhafterweise wird dabei als Startpunkt jeweils ein offenes, noch nicht geflutetes Ventil gewählt. Von diesem offenen, noch nicht gefluteten Ventil aus werden dann alle mit diesem Ventil verbundenen offenen Ventile virtuell geflutet, von welchen aus wiederum alle angrenzenden offenen Ventile geflutet werden, bis keine weiteren angrenzenden offenen Ventile, welch noch nicht geflutet sind, gefunden werden. Am Ende dieses Prozesses ergibt sich dann die von den jeweils gefluteten Verbindungen hergestellte Verbindung zwischen mehreren Quellen und/oder Senken. Weist das System weitere offene, noch nicht geflutete Ventile auf, wird der Prozeß mit einem dieser Ventile als Startpunkt fortgesetzt, um sämtliche durch das System hergestellten Verbindungen festzustellen.

Vorteilhafterweise werden dabei erfindungsgemäß alle ermittelten Verbindungen in einer Verbindungsmatrix eingetragen. Insbesondere kann in eine solche Matrix für alle Kombinationen von Ventilen des Gesamtsystems eingetragen werden, ob durch den aktuellen Schaltzustand der Ventile eine Verbindung zwischen diesen beiden Ventilen besteht oder nicht. Hierbei kann wiederum, wie oben beschrieben, iterativ vorgegangen werden, indem nacheinander alle offenen Ventile von ein oder mehreren Startpunkten aus virtuell geflutet werden und die sich daraus ergebenden Verbindungen in die Verbindungsmatrix eingetragen werden.

Hierdurch muss nur noch die sich ergebende Verbindungsmatrix mit einer Kontrollmatrix verglichen werden, in welcher alle unzulässigen Verbindungen eingetragen sind. Weist die sich ergebende Verbindungsmatrix dabei keinen Eintrag auf, welcher einer unzulässigen Verbindung entsprechen würde, sind die durch den aktuellen und/oder geplanten Zustand der Ventile bereitgestellten Verbindungen zulässig.

Wird dagegen eine unzulässige Verbindung für einen Schaltzustand der Ventile erkannt, so werden Sicherheitsmaßnahmen eingeleitet. Erkennt das Verfahren eine unzulässige Verbindung für einen aktuellen Schaltzustand, so schaltet das System vorteilhafterweise in einen sicheren Zustand. Bei einem Erkennen einer unzulässigen Verbindung, welche sich durch einen geplanten Schaltzustand der Ventile ergeben würde, wird dieser Schaltzustand nicht herbeigeführt.

Hierdurch ergibt sich erfindungsgemäß ein äußerst sicheres Verfahren zur Überwachung der Ventile, durch welches sichergestellt wird, dass keine unzulässigen Flusswege hergestellt werden. Da die unzulässigen Flusswege dabei nur einmal vorgegeben werden müssen, muss das Sicherheitssystem nicht an neu implementierte Prozesse angepaßt werden, und erhält damit die hohe Flexibilität des erfindungsgemäßen Systems.

Die vorliegende Erfindung umfasst dabei weiterhin eine Maschine, insbesondere eine medizinische Behandlungsmaschine mit einer Mehrzahl von Ventilaktoren zur Ansteuerung von Ventilen zur Flußwegsteuerung insbesondere in einem Kassettensystem, mit einer Ventilaktorsteuerung und mit einer Überwachungseinheit zur Überwachung des aktuellen und/oder geplanten Zustands einer Mehrzahl von Ventilen, wobei die Überwachungseinheit den aktuellen und/oder geplanten Schaltzustand der Ventile und die durch den Schaltzustand der Ventile sich ergebenden Verbindungen ermittelt und die sich ergebenden Verbindungen mit einer vorgegebenen Menge von unzulässigen Verbindungen vergleicht. Insbesondere ist die erfindungsgemäße Maschine damit mit einer Steuerung ausgerüstet, welche so programmiert ist, dass auf dieser das erfindungsgemäße Verfahren zur Überwachung der Ventilstellung insbesondere automatisch durchgeführt wird. Dabei wird vorteilhafterweise der Schaltzustand eines Ventils durch den Schaltzustand des zugeordneten Ventilaktors der Maschine bestimmt, so dass zur Überwachung des Schaltzustands der Ventile die Überwachung des Schaltzustands der maschinenseitigen Ventilaktoren herangezogen werden kann. Die Ventile selbst sind dagegen üblicherweise Bestandteil einer Kassette, welche als Einwegteil in die Maschine eingelegt wird.

Durch die erfindungsgemäße Maschine ergibt sich dabei eine flexible Möglichkeit, den Schaltzustand der Ventilaktoren bzw. Ventile und damit die Zulässigketi der aktuellen und/oder geplanten Flußwege sicher zu überwachen.

Vorteilhafterweise erfolgt dabei die Ermittlung der sich durch einen Schaltzustand ergebenden Verbindungen immer dann, wenn sich der Schaltzustand eines Ventils ändert oder ändern soll. Die erfindungsgemäße Maschine überprüft damit immer dann die Zulässigkeit der Verbindungen, wenn ein Ventilaktor angesteuert wird oder angesteuert werden soll.

Vorteilhafterweise werden dabei zur Ermittlung der sich durch einen Schaltzustand ergebenden Verbindungen alle offenen Ventile von einem oder mehreren Startpunkten aus virtuell geflutet. Die erfindungsgemäße Maschine umfasst dabei vorteilhafterweise einen Speicher, in welchem eine Repräsentation der Ventile und der Flußwege abgespeichert ist. Weiterhin umfasst die Maschine vorteilhafterweise eine Recheneinheit, welche auf Grundlage dieser Daten eine virtuelle Flutung durchführt.

Vorteilhafterweise wird dabei als Startpunkt jeweils ein offenes, noch nicht geflutetes Ventil gewählt. Weiterhin vorteilhafterweise werden dabei alle ermittelten Verbindungen in eine Verbindungsmatrix eingetragen. Hierfür weist die erfindungsgemäße Maschine vorteilhafterweise einen Speicher auf, in welchem die ermittelten Verbindungen abgespeichert werden können.

Weiterhin vorteilhafterweise wird dabei die sich ergebende Verbindungsmatrix mit einer Kontrollmatrix verglichen, in welcher alle unzulässigen Verbindungen eingetragen sind. Auch hierfür weist die erfindungsgemäße Maschine vorteilhafterweise einen Speicher auf, in welchem die Kontrollmatrix abgelegt ist. Die Überwachungseinheit vergleicht dann vorteilhafterweise die sich ergebende Verbindungsmatrix mit der abgespeicherten Kontrollmatrix.

Vorteilhafterweise schaltet dabei die Maschine bei Erkennen einer unzulässigen Verbindung bei einem aktuellen Schaltzustand der Ventile in einen sicheren Zustand und/oder führt bei Erkennen einer unzulässigen Verbindung bei einem geplanten Schaltzustand diesen Schaltzustand nicht herbei. Hierdurch wird die Sicherheit der Maschine garantiert.

Vorteilhafterweise weist die erfindungsgemäße Maschine dabei eine Ankoppelfläche zum Ankoppeln einer Kassette auf, wobei die Kassette ventil- und flüssigkeitsführende Kanäle umfasst, und wobei die maschinenseitigen Ventilaktoren zur Herstellung unterschiedlicher Flußwege in der Kassette den Schaltzustand der kassettenseitigen Ventile bestimmen.

Dabei sind sowohl das erfindungsgemäße Verfahren zur Ansteuerung der Ventile als auch das erfindungsgemäße Verfahren zur Überwachung des Zustands der Ventile unabhängig voneinander von großem Vorteil. Für den Fachmann ist jedoch offensichtlich, dass gerade die Kombination des äußerst flexiblen Ansteuerungsverfahrens mit dem ebenso flexiblen Überwachungsverfahren besondere Vorteile insbesondere bei der Implementierung neuer Prozesse ergibt.

Die vorliegende Erfindung umfasst weiterhin eine Maschine, insbesondere eine medizinische Behandlungsmaschine, mit einer Mehrzahl von Ventilaktoren zur Ansteuerung von Ventilen zur Flusswegsteuerung insbesondere in einem Kassettensystem, wobei die Maschine eine Steuerung zur Durchführung eines der oben beschriebenen Verfahren aufweist. Insbesondere ist die erfindungsgemäße Steuerung dabei so programmiert, dass eines oder mehrere der erfindungsgemäßen Verfahren durchgeführt werden. Hierdurch ergeben sich offensichtlich die gleichen Vorteile, wie sie bereits oben bezüglich des Verfahrens beschrieben wurden.

Weiterhin umfasst die vorliegende Erfindung ein Computerprogrammprodukt, insbesondere ein Speichermedium mit einem Computerprogramm, zum Aufspielen auf eine Maschine, insbesondere auf eine medizinische Behandlungsmaschine, mit Befehlen zur Durchführung eines Verfahrens, wie es oben beschrieben wurde. Durch ein solches Computerprogrammprodukt kann eine bestehende Maschine, insbesondere eine bestehende medizinische Behandlungsmaschine, auf welche bisher Verfahren gemäß dem Stand der Technik eingesetzt werden, mittels eines Updates in die Lage versetzt werden, die erfindungsgemäßen Verfahren durchzuführen.

Die vorliegende Erfindung wird nun anhand von Ausführungsbeispielen sowie Zeichnungen näher dargestellt. Dabei zeigen:
- Figur 1:: eine Ventilanordnung, zu deren Ansteuerung und/oder Überwa- chung Ausführungsbeispiele der erfindungsgemäßen Verfahren zum Einsatz kommen, sowie eine Legende für die in den darauf folgenden Zeichnungen verwendeten Symbole,
- Figur 2a:: einen ersten Prozeß, welcher in einem Ausführungsbeispiel des erfindungsgemäßen Verfahrens zur Ansteuerung von Ventilen zum Einsatz kommt,
- Figur 2b:: einen zweiten Prozeß, welcher in dem Ausführungsbeispiel des erfindungsgemäßen Verfahrens zur Ansteuerung von Ventilen zum Einsatz kommt,
- Figur 3:: eine diagrammatische Darstellung des Ausführungsbeispiels des erfindungsgemäßen Verfahrens zur Ansteuerung der Ventile,
- Figur 4:: einen ersten Zustand einer Mehrzahl von Ventilen, welcher durch ein Ausführungsbeispiel des erfindungsgemäßen Verfah- rens zur Überwachung überwacht wird,
- Figur 5:: einen zweiten Zustand einer Mehrzahl von Ventilen, welcher durch das Ausführungsbeispiel des erfindungsgemäßen Verfah- rens zur Überwachung überwacht werden soll,
- Figuren 6a - 6d:: einzelne Schritte des Ausführungsbeispiels des erfindungsge- mäßen Verfahrens zur Überwachung eines Zustands,
- Figur 7:: eine Verbindungsmatrix, welche in dem Ausführungsbeispiel des erfindungsgemäßen Verfahrens zur Überwachung zum Einsatz kommt und dem in Figur 4 gezeigten Zustand einer Mehrzahl von Ventilen entspricht,
- Figur 8:: den Ausgangspunkt für eine Verbindungsmatrix, wie sie durch das Ausführungsbeispiel des erfindungsgemäßen Verfahrens zur Überwachung für den in Figur 5 gezeigten Zustand einer Mehr- zahl von Ventilen erstellt wird und
- Figuren 9a - 9d:: einzelne Schritte bei der Erstellung einer Verbindungsmatrix durch das Ausführungsbeispiels des erfindungsgemäßen Verfah- rens zur Überwachung eines Zustands, welche den in Figuren 6a bis 6d gezeigten Schritten entsprechen.

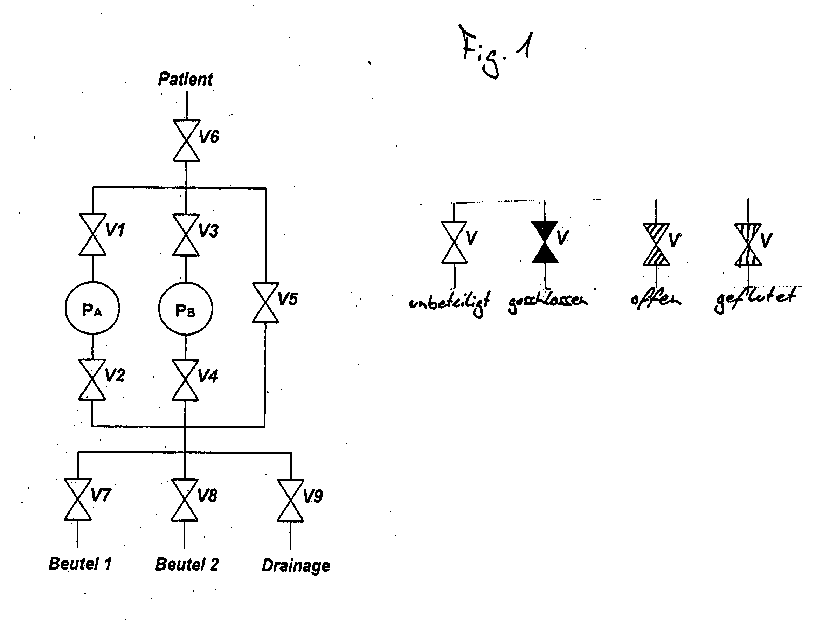

In Figur 1 ist eine Ventilanordnung gezeigt, zu deren Ansteuerung und/oder Überwachung die erfindungsgemäßen Verfahren eingesetzt werden können. Die gezeigten Fluidwege und Ventile sind dabei in einer Einwegkassette integriert, welche in eine erfindungsgemäße Behandlungsmaschine eingelegt und dort mit einer Ankoppelfläche verkoppelt wird. Die Ankoppelfläche der Behandlungsmaschine weist dabei Ventilaktoren auf, welche mit Ventilstellen an der Kassette zusammenwirken und hierdurch Ventile bilden.

Die Fluidwege in der Kassette werden dabei durch flüssigkeitsführende Bereiche gebildet, welche zumindest im Bereich der Ventilstellen flexible Wände aufweisen. Die flexiblen Bereiche werden können von den Ventilaktoren in die flüssigkeitsführenden Bereiche hineingedrückt werden, um diese zu verschließen. Insbesondere besteht die Kassette dabei aus einem Hartteil mit flüssigkeitsführenden Kanälen, welche von einer flexiblen Folie abgedeckt sind. Die Einwegkassette weist neben den Fluidwegen und den Ventilen weiterhin Pumpkammern P_{A} und P_{B} auf, welche ebenfalls als von einer flexiblen Membran abgedeckte flüssigkeitsführende Bereiche ausgebildet sind, wobei die Membran durch die Behandlungsmaschine in die Kassette hineingedrückt und/oder aus dieser herausgezogen werden kann, so dass sich eine Pumpfunktion ergibt.

Bei dem in Figur 1 gezeigten Ventilsystem sind dabei mehrere Ein- bzw. Ausgaänge vorgesehen, welche über die Ventile miteinander und/oder den Pumpkammern verbunden werden können. Insbesondere sind dabei Anschlüsse für Flüssigkeitsbeutel sowie für den Patienten vorgesehen. Jeder der Anschlüsse für externe Leitungen weist dabei ein eigenes Ventil auf. Weiterhin sind an den Ein- und Ausgängen der beiden Pumpkammern P_{A} und P_{B} jeweils Ventile vorgesehen.

Die Pumpkammern stehen damit über Ventile V1, V3 und V6 mit dem Patientenanschluß in Verbindung. Weiterhin stehen die Pumpkammern über Ventile V2, V4 und V7 bis V9 mit den Flüssigkeitsbeuteln in Verbindung. Der Patientenanschluß steht über das Ventil V5 ebenfalls direkt mit den Flüssigkeitsbeuteln in Verbindung. Dabei sind in der in Figur 1 gezeigten Anordnung zwei Anschlüsse für Flüssigkeitsbeutel für Behandlungsflüssigkeiten sowie ein Anschluß für einen Drainagebeutel vorgesehen.

Die beiden Pumpkammern sind dabei parallel zueinander zwischen einer gemeinsamen Leitung, welche zu den Flüssigkeitsbeuteln führt, und einer Leitung, welche zum Patienten führt, angeordnet. Weiterhin ist eine zu den Pumpkammern parallele Leitung mit dem Ventil V5 vorgesehen, über welches eine direkte Verbindung zwischen Patient und Flüssigkeitsbeuteln hergestellt werden kann.

Ausführungsbeispiele der erfindungsgemäßen Verfahren zur Ansteuerung der Ventile V1 bis V9 sowie zu deren Überwachung werden nun anhand der Figuren 1 bis 9 näher beschrieben. Hierzu werden die in Figur 1 gezeigten Symbole für die unterschiedlichen Schaltzustände der Ventile verwendet. Ein weiß ausgefülltes Ventil steht dabei für ein unbeteiligtes Ventil, dessen Schaltzustand bezüglich des entsprechenden Verfahrens unbestimmt ist. Ein schwarz ausgefülltes Ventil steht für ein geschlossenes Ventil, ein mit schräger Schraffur ausgefülltes Ventil steht für ein offenes Ventil. Ein mit vertikaler Schraffur ausgefülltes Ventil steht dagegen für ein offenes Ventil, welches für das Verfahren virtuell geflutet wurde.

In Figur 1 ist dabei eine Ausgangsstellung eines Verfahrens zur Ansteuerung von Ventilen zur Flusswegsteuerung einer medizinischen Behandlungsmaschine gezeigt. Dabei sind zunächst alle Ventile unbeteiligt, das heißt sie können mechanisch offen oder geschlossen sein. Da ein gewisser Anfangsstatus für die Steuerung der Flusswege nötig ist, werden die Ventile im Ausführungsbeispiel initial geschlossen.

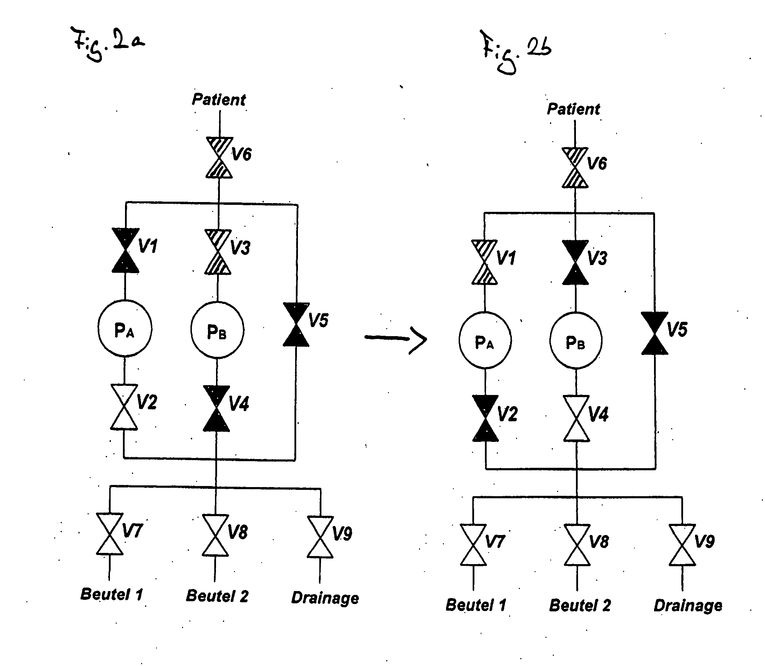

Das Ausführungsbeispiel des erfindungsgemäßen Verfahrens umfasst dabei eine Mehrzahl von Prozessen zur Herstellung von Flusswegen durch die Ansteuerung einer Gruppe von Ventilen, wobei jeder Prozeß während seiner Durchführung die jeweils benötigte Gruppe von Ventilen für sich beansprucht, so dass andere Prozesse diese Ventile nicht ansteuern können. In Figur 2a ist dabei ein erster Prozeß B gezeigt, welcher den Flussweg vom Patienten zur Pumpe P_{B} herstellt. Hierfür sind die Ventile V1, V4 und V5 geschlossen, während die Ventile V3 und V6 geöffnet sind. Alle anderen Ventile bleiben unbeteiligt. Der in Figur 2a gezeigte Prozeß beansprucht damit die Ventile V1, V3 und V4 bis V6 für sich, so dass andere Prozesse den Schaltzustand dieser Ventile nicht ändern können.

In Figur 2b ist nun ein zweiter Prozeß A gezeigt, durch welchen ein Flussweg vom Patienten zur Pumpkammer P_{A} hergestellt werden soll. Hierfür müssen die Ventile V2, V3 und V5 geschlossen und die Ventile V1 und V6 geöffnet werden, während die anderen Ventile unbeteiligt bleiben. Der zweite Prozeß A benötigt damit die Ventile V1 bis V3 sowie V5 und V6. Bis auf das Ventil V2 sind jedoch alle diese Ventile noch vom ersten Prozeß B belegt, so dass der Prozeß A diese nicht ansteuern kann. Insbesondere ist dabei das vom ersten Prozeß belegte Ventil V3 in seiner offenen Stellung, während es für den zweiten Prozeß geschlossen sein müßte. Die vom zweiten Prozeß gestellte Anfrage, ob die zur Herstellung des gewünschten Flussweges benötigten Ventile zur Verfügung stehen, ergibt damit, dass die benötigten Ventile teilweise von einem anderen Prozeß belegt sind, da das in Ventil V3 von dem ersten Prozeß beansprucht wird und in seiner Schaltstellung geändert werden müsste.

Der zweite Prozeß belegt damit zunächst nur die bereits verfügbaren Ventile, welche zur Bereitstellung des Fluidwegs benötigt werden, d.h. das noch nicht beanspruchte Ventil V2 sowie die vom ersten Prozeß beanspruchten Ventile V1, V5 und V6, welche sich bereits im vom zweiten Prozeß benötigten Schaltzustand befinden, und stellt eine Anfrage an den ersten Prozeß, damit dieser die noch benötigten Ventile freigibt. Der erste Prozeß entscheidet dann, ob und wann er die benötigten Ventile, d.h. das Ventil V3, welches umgeschaltet werden müßte, freigibt. Der zweite Prozeß muss solange warten. Die Entscheidung des ersten Prozesses über die Freigabe der angeforderten Ventile kann dabei durch eigene Prozesskriterien wie z. B. die Pumpenposition, oder durch äußere Umstände wie z. B. eine höhere Priorisierung des anderen Prozesses, beeinflußt werden. Entscheidend ist hierbei jedoch, dass nur der erste Prozeß die durch ihn benutzten Ventile freigeben kann. Hierdurch ist ein ebenso sicheres wie koordiniertes Zusammenspiel der Prozesse gewährleistet. Erfindungsgemäß kann demnach nur der Prozeß, welcher ein bestimmtes Ventil aktuell beansprucht hat, dieses auch wieder freigegeben.

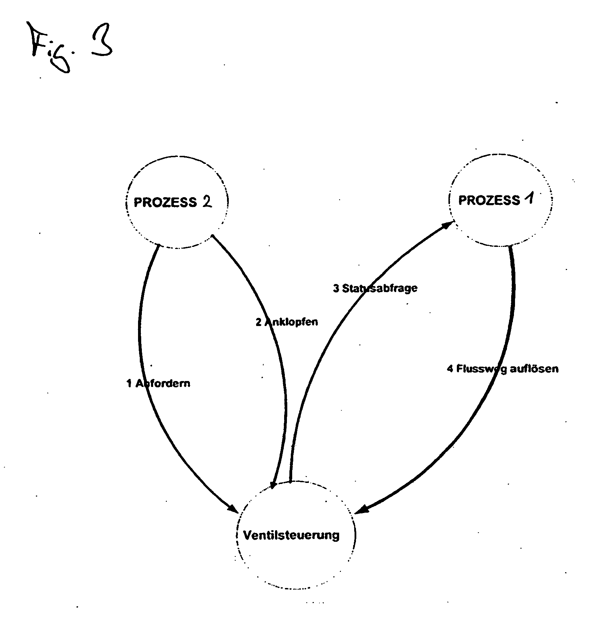

Der Ablauf des Ausführungsbeispiels des erfindungsgemäßen Verfahrens zur Ansteuerung der Ventile wird dabei noch einmal anhand des in Figur 3 gezeigten Ablaufschemas näher dargestellt. Der Prozeß 1 hat dabei einen gewissen Flussweg hergestellt und beansprucht hierzu eine erste Gruppe von Ventilen. Der zweite Prozeß 2 fragt nun zunächst die Verfügbarkeit einer zweiten Gruppe von Ventilen ab, durch deren Ansteuerung ein zweiter Flussweg hergestellt werden soll. Diese Abfrage der verfügbaren Ventile stellt gleichzeitig eine Anforderung an die zentrale Ventilsteuerung dar, diese Ventile dem zweiten Prozeß zuzuordnen. Dabei kann der zweite Prozeß durch seine Abfrage jedoch lediglich jene Ventile für sich beanspruchen, welche verfügbar sind. Für die nicht verfügbaren abgefragten Ventile kann der zweite Prozeß lediglich ein Beanspruchungsrecht von der Ventilsteuerung erhalten, so dass er diese Ventile für sich beanspruchen kann, wenn diese freigegeben werden. Hierdurch kann verhindert werden, dass ein dritter Prozeß die vom zweiten Prozeß benötigten aber noch nicht freigegebenen Ventile für sich beansprucht, wenn diese freigegeben werden.

Bezüglich der abgefragten aber nicht verfügbaren Ventile klopft der zweite Prozeß nun über die Ventilsteuerung beim ersten Prozeß an, und fordert diesen damit auf, die benötigten Ventile freizugeben. Der Prozeß 1 kann hier z. B. mit Hilfe einer Statusabfrage (Polling) detektieren, dass die Ventile benötigt werden und der von Prozeß 1 gebildete Flussweg aufgelöst werden soll. Der erste Prozeß kann nun frei entscheiden, wie zu verfahren ist. Insbesondere entscheidet nur der erste Prozeß über die Anfrage, ob die vom zweiten Prozeß angefragten und vom ersten Prozeß beanspruchten Ventile freigegeben werden oder nicht. Gibt der erste Prozeß die Ventile frei, so meldet er dies der zentralen Ventilsteuerung. Hierdurch sind alle für den zweiten Prozeß benötigten Ventile verfügbar, so dass dieser durchgeführt werden kann. Der zweite Prozeß beansprucht nun seinerseits die entsprechende Gruppe von Ventilen, um den gewünschten Flussweg bereitzustellen. Dementsprechend kann nun auch nur der zweite Prozeß bei einer weiteren Anfrage eines anderen Prozesses entscheiden, inwieweit die von ihm beanspruchten Ventile wieder freigegeben werden.

Durch das erfindungsgemäße Verfahren zur Ansteuerung der Ventile zur Flusswegsteuerung ergibt sich somit eine äußerst flexible Ansteuerung der Ventile, durch welche sämtliche Ventile des Systems optimal genutzt werden. Die Ventile stellen dabei eine Ressource für die zu bildenden Flusswege dar, da ein Flussweg durch eine Anordnung von geöffneten und geschlossenen Ventilen beschrieben wird. Ein Flussweg ist daher erst dann ausführbar, wenn alle Ressourcen zur Verfügung stehen, das heißt wenn alle an dem Flussweg beteiligten Ventile für den entsprechenden Prozeß zur Verfügung stehen.

Die erfindungsgemäße Ventilsteuerung erlaubt dabei die effektive Planung der zur Verfügung stehenden Ressourcen, indem sie den einzelnen Prozessen verschiedene Funktionalitäten zur Verfügung stellt: Ein Prozeß zur Bereitstellung eines Flußwegs kann dabei Ressourcen anfordern, das heißt zur Verfügung stehende Ventile für sich beanspruchen, wodurch sie anderen Prozessen nicht mehr zur Verfügung stehen, oder für sich beanspruchte Ventile wieder freigeben, wodurch sie anderen Prozessen wieder zur Verfügung stehen. Weiterhin können einzelne Prozesse die Auflösung eines anderen Flussweges anfragen (Anklopfen), um so benötigte Ventile für sich beanspruchen zu können. Ebenso können Prozesse abfragen, ob der durch sie hergestellte Flussweg aufgelöst werden soll (Flusswegstatus), z. B. weil andere Prozesse eine höhere Priorität besitzen. Die Entscheidung über die Auflösung des Flusswegs bzw. die Freigabe der daran beteiligten Ventile trifft jedoch immer der Prozess, welcher die freizugebenden Ressourcen benutzt.

Hierdurch ergibt sich eine einfach zu implementierende Möglichkeit, mehrere Prozesse, z. B. zur Ansteuerung mehrerer Pumpen, miteinander zu synchronisieren, ohne dass die Sicherheit des Gesamtsystems beeinträchtigt würde.

Für die beschriebene flexible Flusswegplanung wird auch die Logik des Sicherheitssystems der erfindungsgemäßen Behandlungsmaschine invertiert. Es werden dabei nicht mehr alle erlaubten Flusswege hinterlegt, die dann bei Änderung oder Neueinführung eines Verfahrens überarbeitet werden müßten, sondern es werden für die Gesamtanordnung der Ventile, Pumpen und Anschlüsse einmalig alle verbotenen Wege ermittelt und hinterlegt.

Die vorliegende Erfindung weist hierfür ein Verfahren zur Überwachung des Zustands der Ventile bei der Flusswegsteuerung auf. Hierzu werden alle Ventilstati überwacht. Ändert sich ein Ventilstatus oder plant ein Prozeß eine Ventilstatusänderung, so werden alle sich durch den aktuellen bzw. geplanten Schaltzustand der Ventile ergebenden Verbindungen ermittelt. Die dabei ermittelten Verbindungen werden dann mit der vorgegebenen Menge von unzulässigen Verbindungen verglichen, um unzulässige Verbindungen und damit unzulässige Schaltzustände zu erkennen.

Zur Ermittelung der Verbindungen, welche sich aus einem Schaltzustand der Ventile ergeben, werden alle offenen Ventile von einem oder mehreren Standpunkten aus virtuell geflutet und das Ergebnis der Flutung wird in einer Verbindungsmatrix eingetragen. Jetzt kann aus der so entstandenen Verbindungsmatrix direkt überprüft werden, ob ein mit Schutzsystem hinterlegter verbotener Weg verwendet wird bzw. verwenden soll.

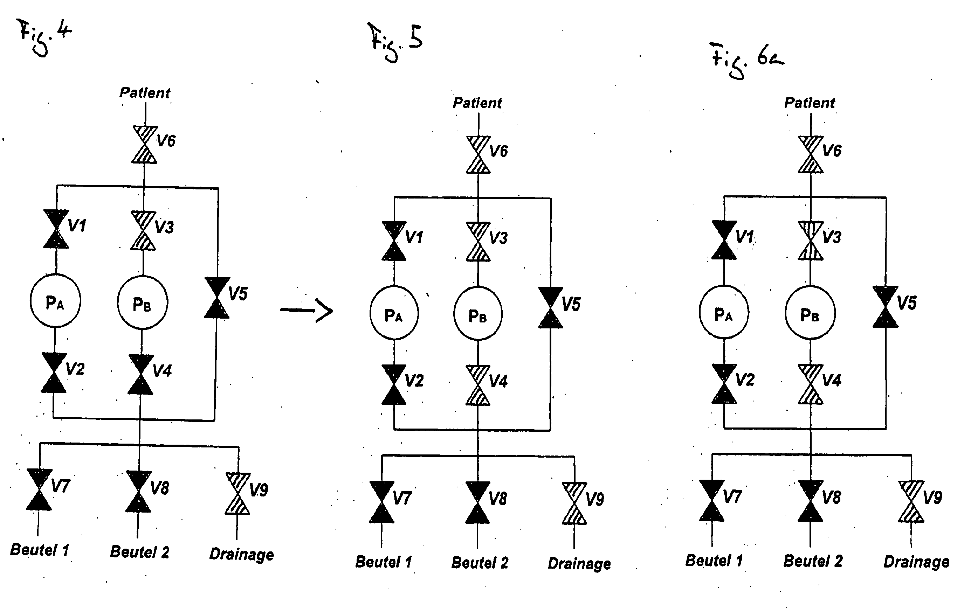

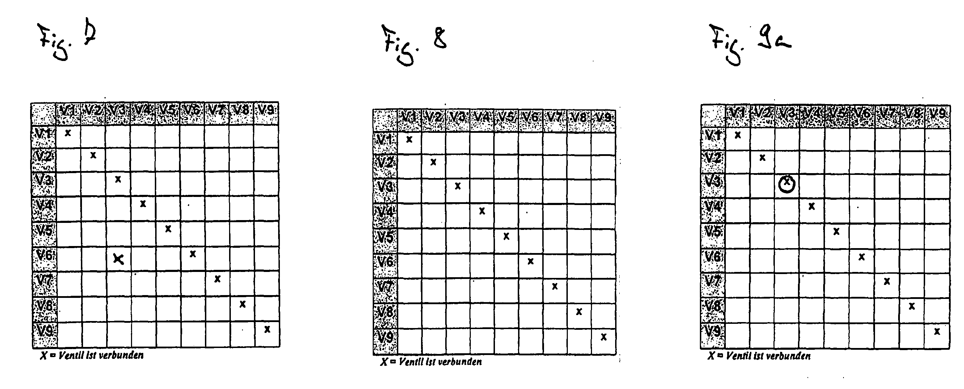

In Figur 4 ist dabei ein erster Schaltzustand der Ventile gezeigt, bei welchem die Ventile V3, V6 und V9 geöffnet und die übrigen Ventile geschlossen sind. Hierdurch ist der Patient mit der Pumpkammer P_{B} verbunden. Dieser Zustand entspricht der in Figur 7 gezeigten Verbindungsmatrix, bei welcher lediglich eine Verbindung zwischen dem Ventil V3 und dem Ventil V6 vorliegt. Das ebenfalls offene Ventil V9 ist mit keinen weiteren offenen Ventilen verbunden, so dass dieses offene Ventil nicht weiter in die Verbindungsmatrix eingeht.

Soll nun aus dem in Figur 4 gezeigten Schaltzustand in den in Figur 5 gezeigten Schaltzustand gewechselt werden, indem das Ventil V4 geöffnet wird, so überprüft das erfindungsgemäße Verfahren zur Überwachung des geplanten Zustands der Ventile, ob sich hierdurch eine unzulässige Verbindung ergibt.

Hierbei wird zunächst von einer noch leeren Verbindungsmatrix ausgegangen, wie sie in Figur 8 gezeigt ist. Durch virtuelles Fluten aller offenen Ventile werden nun schrittweise alle Verbindungen, welche sich durch den in Figur 5 gezeigten Zustand der Ventile ergeben, eingetragen. Verbindungen zwischen zwei Ventilen Vx und Vy werden dabei in der Verbindungsmatrix durch einen Eintrag in dem der Kombination der Ventile Vx und Vy zugeordneten Feld der Matrix gekennzeichnet. Der Übersicht halber werden dabei in den in Figuren 7 bis 9 gezeigten Verbindungsmatrizen Verbindungen lediglich in der linken unteren Hälfte der Matrix dargestellt. Die Felder in der rechten oberen Hälfte sind hierzu äquivalent.

Das Verfahren zur Ermittlung der sich aus einem Schaltzustand der Ventile ergebenden Verbindungen mittwls einer virtuellen Flutung wird nun anhand der Figuren 6a bis 6d sowie der zugehörigen sich ergebenden Verbindungsmatrizen 9a bis 9d beschrieben.

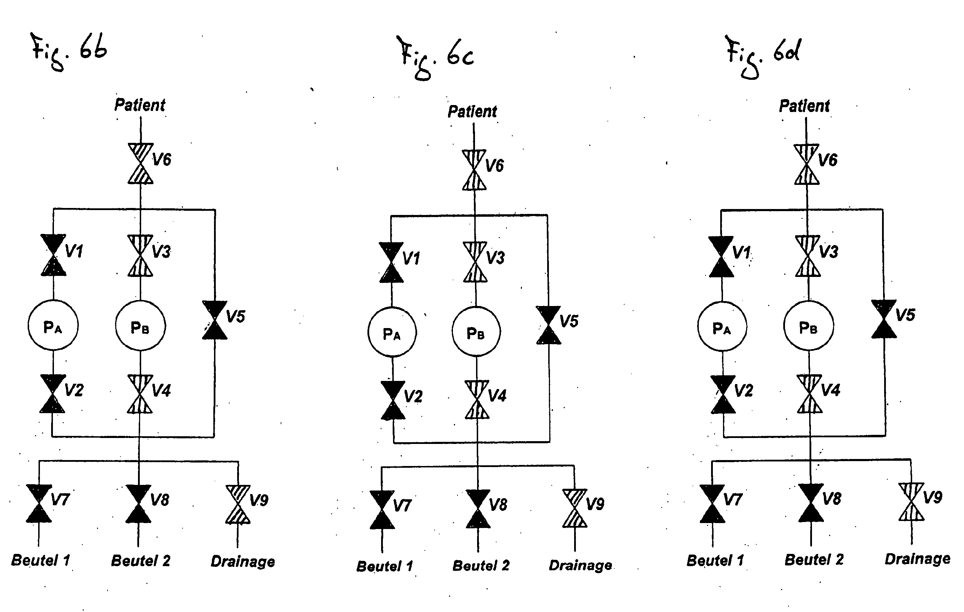

Hierzu wird in dem in Figur 5 gezeigten Zustand der Ventile zunächst ein noch nicht geflutetes, offenes Ventil ausgewählt und virtuell geflutet. Im gezeigten Beispiel wird, wie in Figur 6a dargestellt, zunächst das Ventil V3 geflutet. Die entsprechende Auswahl ist dabei in Figur 9a durch die Umkreisung des dem Ventil V3 selbst zugeordneten Feldes in der Verbindungsmatrix dargestellt. Von dem Ventil V3 ausgehend werden nun, wie in Figuren 6b und 6c dargestellt, alle an das als Startpunkt ausgewählte Ventil angrenzenden offenen Ventile geflutet. In Figur 6b wird daher das Ventil V4, in Figur 6c das Ventil V6 geflutet.

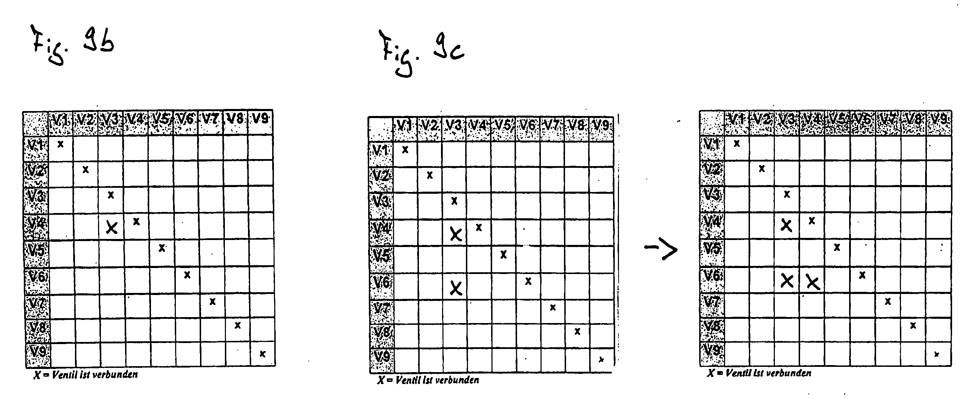

Die sich durch diese Flutung ergebenden Verbindungen werden dabei in der Verbindungsmatrix abgelegt. So wird zunächst, wie in Figur 9b dargestellt, die durch Flutung des Ventils V4 hergestellte Verbindung zwischen den Ventilen V3 und V4 durch eine Markierung des entsprechenden Feldes in der Verbindungsmatrix abgelegt. Durch die in Figur 6c gezeigte Flutung des ebenfalls an das Ventil V3 angrenzenden Ventils V6 ergibt sich nun zunächst eine Verbindung zwischen den Ventilen V3 und V6, welche zunächst in der ersten in Figur 9c dargestellten Verbindungsmatrix eingetragen wird. Das System überprüft nun, ob sich durch diese neue Verbindung weitere Verbindungen zwischen Ventilen ergeben. Im Ausführungsbeispiel ergibt sich dabei durch die Verbindung des Ventils V3 mit dem Ventil V6 einerseits und dem Ventil V4 andererseits eine Verbindung zwischen dem Ventil V6 und dem Ventil V4, welche nun in einem zweiten Schritt, wie in der zweiten in Figur 9c dargestellt, in die Verbindungsmatrix eingetragen wird.

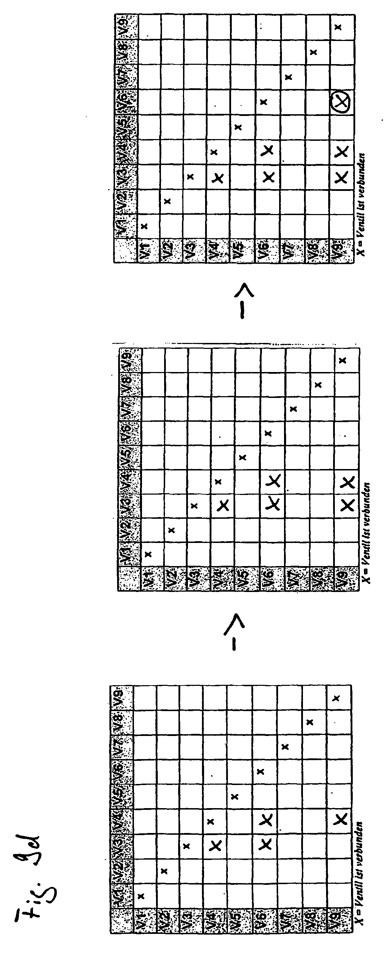

Das Verfahren fährt nun damit fort, weitere an die bereits gefluteten Ventile angrenzenden offenen Ventile zu fluten. An das Ventil V6 grenzt dabei kein weiteres offenes ungeflutetes Ventil an, so dass das Verfahren hier abbricht. An das geflutete Ventil V4 grenzt dagegen das in Figur 6c noch ungeflutete offene Ventil V9 an, welches nun im nächsten Schritt, welcher in Figur 6d gezeigt ist, ebenfalls geflutet wird. Hierdurch ergibt sich zunächst eine Verbindung zwischen dem Ventil V4 und dem Ventil V9, welche nun in einem ersten Schritt in die Verbindungsmatrix eingetragen wird. Dies ist in der ersten in Figur 9d gezeigten Verbindungsmatrix dargestellt. Durch die bereits vorhandene Verbindung zwischen dem Ventil V4 und V3 ergibt sich hierdurch jedoch auch die neue Verbindung zwischen dem Ventil V9 und V3, welche nun in einem zweiten Schritt in die Verbindungsmatrix eingetragen wird. Weiterhin ergibt sich durch die bereits vorhandene Verbindung zwischen dem Ventil V6 mit dem Ventil V3 bzw. dem Ventil V4 eine neue Verbindung zwischen dem Ventil V9 und dem Ventil V6, welche nun im letzten Schritt ebenfalls in die Verbindungsmatrix eingetragen wird.

Nachdem sämtliche mit dem als Startpunkt gewählten Ventil verbundenen offenen Ventile geflutet wurden und sämtliche hierdurch erzeugten Verbindungen in die Verbindungsmatrix eingetragen wurden, überprüft das System, ob weitere, noch ungeflutete offene Ventile vorhanden sind. Findet das System ein solches Ventil, so wird dieses als Startpunkt für eine weitere virtuelle Flutung, welche nach dem gleichen Muster abläuft, gewählt. Das System wiederholt diesen Vorgang so lange, bis alle offenen Ventile geflutet wurden.

In der sich aus dieser virtuellen Flutung ergebenden Verbindungsmatrix sind nunmehr alle Verbindungen eingetragen, welche sich durch den untersuchten Zustand der Ventile ergeben.

Die Verbindungsmatrix wird nun mit einer Kontrollmatrix verglichen, in welcher alle unzulässigen Verbindungen eingetragen sind. Z. B. enthält eine solche Kontrollmatrix die unzulässige Verbindung zwischen den Ventilen V9 und V6, durch welche eine Verbindung zwischen dem Patienten und der Drainage hergestellt würde. Das erfindungsgemäße Verfahren erkennt damit anhand der für den in Figur 5 gezeigten Zustand ermittelten Verbindungsmatrix, welche ganz rechts in Figur 9d gezeigt ist, dass durch diesen Zustand eine unzulässige Verbindung hergestellt würde. Dementsprechend verhindert das erfindungsgemäße Sicherheitssystem, dass in diesen Zustand geschaltet wird. Wird vom Sicherheitssystem dagegen ein aktueller Schaltzustand überprüft und festgestellt, dass hierbei eine unzulässige Verbindung vorliegt, so schaltet das System automatisch in einen sicheren Zustand.

Dabei müssen in der Kontrollmatrix nur unzulässige Verbindungen zwischen den Anschlüssen angegeben werden, d.h. zwischen den Ventilen V6 bis V9. Wie diese Verbindungen realisiert sind, ist für die Beurteilung dagegen unerheblich. Damit müssen nicht mehr alle zulässigen Schaltzustände aller Ventile im Sicherheitssystem abgelegt werden, sondern nur noch die unzulässigen Verbindungen zwischen Anschlüssen, während das System selbstständig überprüft, ob ein konkreter Schaltzustand zu einer solchen unzulässigen Verbindung führt. Denkbar wäre es, alternativ zu den unzulässigen Verbindungen zwischen den Anschlüssen die zulässigen Verbindungen zwischen den Anschlüssen im Sicherheitssystem abzulegen. Auch dann müßten nicht mehr alle zulässigen Schaltzustände im System abgespeichert werden. Da aber erheblich mehr zulässige als unzulässige Verbindungen vorliegen, ist das Abspeichern der unzulässigen Verbindungen effektiver.

Die vorliegende Erfindung umfasst dabei neben den gezeigten Ausführungsbeispielen der Verfahren weiterhin eine Behandlungsmaschine, insbesondere für die Peritonealdialyse, in deren Steuerung die entsprechenden Verfahren implementiert sind. Weiterhin umfasst die vorliegende Erfindung ein Computerprogrammprodukt, insbesondere ein Speichermedium, auf welchem eine entsprechende Steuerungssoftware gespeichert ist, welche auf entsprechende Behandlungsmaschinen aufgespielt werden kann, um die erfindungsgemäßen Verfahren dort zu implementieren.

## Patentansprüche

1. Verfahren zur Ansteuerung von Ventilen zur Flußwegsteuerung, insbesondere in einer medizinischen Behandlungsmaschine, mit einer Mehrzahl von Prozessen zur Herstellung von Flußwegen durch Ansteuerung einer Gruppe von Ventilen,
**dadurch gekennzeichnet,**
**dass** jeder Prozeß eine zu seiner Durchführung geeignete Gruppe von Ventilen für sich beansprucht, so dass andere Prozesse den Schaltzustand dieser Ventile nicht ändern können, und dass jeder Prozeß selbst über die Freigabe der durch ihn beanspruchten Ventile entscheidet.

2. Verfahren nach Anspruch 1, wobei jeder Prozeß nach internen und/oder externen Prozeßkriterien über die Freigabe der durch ihn beanspruchten Ventile entscheidet, wobei insbesondere jeder Prozeß nach seiner Beendigung die beanspruchte Gruppe von Ventilen freigibt.

3. Verfahren nach einem der vorangegangenen Ansprüche, wobei ein Prozeß je nach Verfügbarkeit der Ventile unterschiedliche Gruppen von Ventilen beanspruchen kann, wobei wahlweise jeder Prozeß vor seiner Durchführung die Verfügbarkeit einer zu seiner Durchführung geeigneten Gruppe an Ventilen abfragt und wobei wiederum wahlweise ein Prozeß durchgeführt wird, wenn eine geeignete Gruppe an Ventilen verfügbar ist, wobei der Prozeß diese Gruppe an Ventilen für sich beansprucht.

4. Verfahren nach Anspruch 3, wobei die Abfrage eines nicht verfügbaren Ventils durch einen zweiten Prozeß zu einer Aufforderung an den ersten Prozeß, welcher dieses Ventil beansprucht hat, führt, dieses Ventil freizugeben, wobei nur der erste Prozeß über diese Aufforderung entscheidet, wobei der abfragende Prozeß mit der Abfrage vorzugsweise alle abgefragten und verfügbaren Ventile für sich beansprucht, und wobei der abfragende Prozeß besonders bevorzugt mit der Abfrage für alle abgefragten aber nicht verfügbaren Ventile ein Beanspruchungsrecht erhält, so dass er diese Ventile für sich beanspruchen kann, wenn diese freigegeben wurden.

5. Verfahren nach einem der vorangegangenen Ansprüche, mit einem zentralen Ventilzuordnungsprozeß, bei welchem alle anderen Prozesse die benötigten Ventile beanspruchen, freigeben und/oder anfragen.

6. Verfahren nach einem der vorangegangenen Ansprüche mit den Schritten:
- Beanspruchen einer ersten Gruppe von Ventilen zur Durchführung eines ersten Prozesses, bei welchem ein erster Flußweg durch Ansteuerung der zugehörigen ersten Gruppe an Ventilen hergestellt wird;
- Abfrage der Verfügbarkeit einer zweiten Gruppe an Ventilen zur Durchführung eines zweiten Prozesses, bei welchem ein zweiter Flußweg durch Ansteuerung der zugehörigen zweiten Gruppe an Ventilen hergestellt werden soll;
- Durchführen des zweiten Prozesses, wenn alle benötigten Ventile verfügbar sind;
wobei die Abfrage durch den zweiten Prozeß von Ventilen, welche nicht verfügbar sind, zu einer Aufforderung an den ersten Prozeß führt, diese Ventile freizugeben, wobei nur der erste Prozeß über diese Anfrage entscheidet.

7. Maschine, insbesondere medizinische Behandlungsmaschine, mit einer Mehrzahl von Ventilaktoren zur Ansteuerung von Ventilen zur Flußwegsteuerung, insbesondere in einem Kassettensystem, mit einer Ventilaktorsteuerung zur Durchführung einer Mehrzahl von Prozessen zur Herstellung von Flußwegen durch Ansteuerung einer Gruppe von Ventilaktoren,
**dadurch gekennzeichnet,**
**dass** die Ventilaktorsteuerung so ausgeführt ist, dass jeder Prozeß eine zu seiner Durchführung geeignete Gruppe von Ventilaktoren für sich beansprucht, so dass andere Prozesse den Schaltzustand dieser Ventilaktoren nicht ändern können, und jeder Prozeß selbst über die Freigabe der durch ihn beanspruchten Ventilaktoren entscheidet.

8. Maschine nach Anspruch 7, wobei jeder Prozeß nach internen und/oder externen Prozeßkriterien über die Freigabe der durch ihn beanspruchten Ventilaktoren entscheidet, wobei jeder Prozeß vorzugsweise nach seiner Beendigung die beanspruchte Gruppe von Ventilaktoren freigibt, wobei weiter bevorzugt ein Prozeß je nach Verfügbarkeit der Ventilaktoren unterschiedliche Gruppen von Ventilaktoren beanspruchen kann und wobei besonders bevorzugt jeder Prozeß vor seiner Durchführung die Verfügbarkeit einer zu seiner Durchführung geeigneten Gruppe an Ventilaktoren abfragt, wobei wahlweise ein Prozeß durchgeführt wird, wenn eine geeignete Gruppe an Ventilaktoren verfügbar ist, wobei der Prozeß diese Gruppe an Ventilaktoren für sich beansprucht.

9. Maschine nach Anspruch 8, wobei die Abfrage eines nicht verfügbaren Ventilaktors durch einen zweiten Prozeß zu einer Aufforderung an den ersten Prozeß, welcher diesen Ventilaktor beansprucht hat, führt, diesen Ventilaktor freizugeben, wobei nur der erste Prozeß über diese Aufforderung entscheidet, wobei der abfragende Prozeß mit der Abfrage vorzugsweise alle abgefragten und verfügbaren Ventilaktoren für sich beansprucht.

10. Maschine nach Anspruch 9, wobei der abfragende Prozeß mit der Abfrage für alle abgefragten aber nicht verfügbaren Ventilaktoren ein Beanspruchungsrecht erhält, so dass er diese Ventilaktoren für sich beanspruchen kann, wenn diese freigegeben wurden.

11. Maschine nach einem der Ansprüche 7 bis 10, wobei die Ventilaktorsteuerung einen zentralen Ventilzuordnungsprozeß umfaßt, bei welchem alle anderen Prozesse die benötigten Ventilaktoren beanspruchen, freigeben und/oder anfragen.

12. Maschine nach einem der Ansprüche 7 bis 11 mit einer Ventilaktorsteuerung, in welcher folgende Schritte durchgeführt werden:
- Beanspruchen einer ersten Gruppe von Ventilaktoren zur Durchführung eines ersten Prozesses, bei welchem ein erster Flußweg durch Ansteuerung der zugehörigen ersten Gruppe an Ventilen hergestellt wird;
- Abfrage der Verfügbarkeit einer zweiten Gruppe an Ventilaktoren zur Durchführung eines zweiten Prozesses, bei welchem ein zweiter Flußweg durch Ansteuerung der zugehörigen zweiten Gruppe an Ventilen hergestellt werden soll;
- Durchführen des zweiten Prozesses, wenn alle benötigten Ventilaktoren verfügbar sind;
wobei die Abfrage durch den zweiten Prozeß von Ventilaktoren, welche nicht verfügbar sind, zu einer Aufforderung an den ersten Prozeß führt, diese Ventilaktoren freizugeben, wobei nur der erste Prozeß über diese Anfrage entscheidet.

13. Maschine nach einem der Ansprüche 7 bis 12 mit einer Ankoppelfläche zum Ankoppeln einer Kassette, welche Ventile und flüssigkeitsführende Kanäle umfaßt, wobei die maschinenseitigen Ventilaktoren zur Herstellung unterschiedlicher Flußwege in der Kassette den Schaltzustand der kassettenseitigen Ventile bestimmen.

14. Verfahren zur Überwachung des aktuellen und/oder geplanten Zustands einer Mehrzahl von Ventilen bei der Flußwegsteuerung insbesondere in einer medizinischen Behandlungsmaschine,
**dadurch gekennzeichnet,**
**dass** das Verfahren folgende Schritte aufweist:
Ermitteln des aktuellen und/oder geplanten Schaltzustands der Ventile,
Ermitteln der durch den Schaltzustand der Ventile sich ergebenden Verbindungen,
Vergleichen der sich ergebenden Verbindungen mit einer vorgegebenen Menge von unzulässigen Verbindungen.

15. Verfahren nach Anspruch 14, wobei die Ermittlung der sich durch einen Schaltzustand ergebenden Verbindungen immer dann erfolgt, wenn sich der Schaltzustand eines Ventils ändert oder ändern soll, wobei zur Ermittlung der sich durch einen Schaltzustand ergebenden Verbindungen vorzugsweise alle offenen Ventile von einem oder mehreren Startpunkten aus virtuell geflutet werden, wobei besonders bevorzugt als Startpunkt jeweils ein offenes, noch nicht geflutetes Ventil gewählt wird, wobei weiter bevorzugt alle ermittelten Verbindungen in einer Verbindungsmatrix eingetragen werden und wobei besonders bevorzugt die sich ergebende Verbindungsmatrix mit einer Kontrollmatrix verglichen wird, in welcher alle unzulässigen Verbindungen eingetragen sind.

16. Verfahren nach einem der Ansprüche 14 oder 15, wobei bei Erkennen einer unzulässigen Verbindung bei einem aktuellen Schaltzustand der Ventile das System in einen sicheren Zustand schaltet und/oder bei Erkennen einer unzulässigen Verbindung bei einem geplanten Schaltzustand dieser Schaltzustand nicht herbeigeführt wird.

17. Maschine, insbesondere medizinische Behandlungsmaschine, mit einer Mehrzahl von Ventilaktoren zur Ansteuerung von Ventilen zur Flußwegsteuerung insbesondere in einem Kassettensystem, mit einer Ventilaktorsteuerung und mit einer Überwachungseinheit zur Überwachung des aktuellen und/oder geplanten Zustands einer Mehrzahl von Ventilen,
**dadurch gekennzeichnet,**
**dass** die Überwachungseinheit den aktuellen und/oder geplanten Schaltzustand der Ventile und die durch den Schaltzustand der Ventile sich ergebenden Verbindungen ermittelt und die sich ergebenden Verbindungen mit einer vorgegebenen Menge von unzulässigen Verbindungen vergleicht.

18. Maschine nach Anspruch 17, wobei die Ermittlung der sich durch einen Schaltzustand ergebenden Verbindungen immer dann erfolgt, wenn sich der Schaltzustand eines Ventils ändert oder ändern soll, wobei bevorzugt zur Ermittlung der sich durch einen Schaltzustand ergebenden Verbindungen alle offenen Ventile von einem oder mehreren Startpunkten aus virtuell geflutet werden, wobei weiter bevorzugt als Startpunkt jeweils ein offenes, noch nicht geflutetes Ventil gewählt wird, wobei wahlweise alle ermittelten Verbindungen in einer Verbindungsmatrix eingetragen werden und wobei die sich ergebende Verbindungsmatrix bevorzugt mit einer Kontrollmatrix verglichen wird, in welcher alle unzulässigen Verbindungen eingetragen sind.

19. Maschine nach einem der Ansprüche 17 oder 18, wobei die Maschine bei Erkennen einer unzulässigen Verbindung bei einem aktuellen Schaltzustand der Ventile in einen sicheren Zustand schaltet und/oder bei Erkennen einer unzulässigen Verbindung bei einem geplanten Schaltzustand diesen Schaltzustand nicht herbeiführt.

20. Maschine nach einem der Ansprüche 17 bis 19 mit einer Ankoppelfläche zum Ankoppeln einer Kassette, welche Ventile und flüssigkeitsführende Kanäle umfaßt, wobei die maschinenseitigen Ventilaktoren zur Herstellung unterschiedlicher Flußwege in der Kassette den Schaltzustand der kassettenseitigen Ventile bestimmen.

21. Computerprogrammprodukt, insbesondere Speichermedium mit einem Computerprogramm, zum Aufspielen auf eine Maschine, insbesondere eine medizinische Behandlungsmaschine, mit Befehlen zur Durchführung eines Verfahrens nach einem der Ansprüche 1 bis 6 und 14 bis 16.

## Claims

1. A method for the control of valves for flow path control, in particular in a medical treatment machine, having a plurality of processes for the establishing of flow paths by controlling a group of valves,
**characterized in that**
each process claims a group of valves suitable for its carrying out for itself so that other processes cannot change the switching state of these valves; and that each process itself makes a decision on the release of the valves claimed by it.

2. A method in accordance with claim 1, wherein each process makes a decision on the release of the valves claimed by it in dependence on internal and/or external process criteria, with, in particular, each process releasing the claimed group of valves after its ending.

3. A method in accordance with one of the preceding claims, wherein a process can claim different groups of valves depending on the availability of the valves, with each process selectively polling at the valves the availability of a group of valves suitable for its carrying out before its carrying out and with a process again being carried out selectively when a suitable group of valves is available, with the process claiming this group of valves for itself.

4. A method in accordance with claim 3, wherein the polling of a non-available valve by a second process results in a request to the first process claiming this valve to release this valve, with only the first process making a decision on this request, with the polling process preferably claiming all polled and available valves for itself on the polling and with the polling process particularly preferably receiving a claim right for all polled but not available valves on the polling so that it can claim these valves for itself when they have been released.

5. A method in accordance with one of the preceding claims having a central valve association process in which all the other processes claim, release and/or poll the required valves.

6. A method in accordance with one of the preceding claims having the steps:
- claiming a first group of valves for the carrying out of a first process in which a first flow path is established by control of the associated first group of valves;
- polling the availability of a second group of valves for the carrying out of a second process in which a second flow path should be established by control of the associated second group of valves;
- carrying out the second process when all required valves are available;
with the polling by the second process of valves which are not available resulting in a request to the first process to release these valves, with only the first process making a decision on this request.

7. A machine, in particular a medical treatment machine, having a plurality of valve actuators for the control of valves for the flow path control, in particular in a cassette system, with a valve actuator control for the carrying out of a plurality of processes for establishing flow paths by controlling a group of valve actuators,
**characterized in that**
the valve actuator control is configured such that each process claims a group of valve actuators suitable for its carrying out for itself so that other processes cannot change the switching state of these valve actuators and each process itself makes a decision on the release of the valve actuators claimed by it.

8. A machine in accordance with claim 7, wherein each process makes a decision on the release of the valve actuators claimed by it in dependence on internal and/or external process criteria, with each process preferably releasing the claimed group of valve actuators after its ending, with a process further preferably being able to claim different groups of valve actuators in dependence on the availability of the valve actuators, and with each process particularly preferably polling the availability of a group of valve actuators suitable for its carrying out before its carrying out, with a process selectively being carried out when a suitable group of valve actuators is available, with the process claiming this group of valve actuators for itself.

9. A machine in accordance with claim 8, wherein the polling of a non-available valve actuator by a second process results in a request to the first process claiming this valve actuator to release this valve actuator, with only the first process making a decision on this request, with the polling process claiming all polled and available valve actuators for itself on the polling.

10. A machine in accordance with claim 9, wherein the polling process receives a claiming right for all polled but not available valve actuators on the polling so that it can claim these valve actuators for itself when they have been released.

11. A machine in accordance with one of the claims 7 to 10, wherein the valve actuator control includes a central valve association process in which all the other processes claim, release and/or poll the required valve actuators.

12. A machine in accordance with one of the claims 7 to 11 having a valve actuator control in which the following steps are carried out:
- claiming a first group of valve actuators for the carrying out of a first process in which a first flow path is established by control of the associated first group of valves;
- polling the availability of a second group of valve actuators for the carrying out of a second process in which a second flow path should be established by control of the associated second group of valves;
- carrying out the second process when all required valve actuators are available;
with the polling by the second process of valve actuators which are not available resulting in a request to the first process to release these valve actuators, with only the first process making a decision on this request.

13. A machine in accordance with one of the claims 7 to 12 having a coupling surface for the coupling of a cassette which comprises valves and liquid conducting passages, wherein the valve actuators on the machine side determine the switching state of the valves on the cassette side to establish different flow paths in the cassette.

14. A method for the monitoring of the then current and/or scheduled state of a plurality of valves in the flow path control, in particular in a medical treatment machine,
**characterized in that**
the method includes the following steps:
determining the then current and/or scheduled switching state of the valves;
determining the connections resulting from the switching state of the valves;
comparing the resulting connections with a predetermined number of non-permitted connections.

15. A method in accordance with claim 14, wherein the determination of the connections resulting from a switching state always takes place when the switching state of a valve changes or should change, with preferably all the open valves being virtually flooded, starting from one or more starting points, for the determination of the connections resulting from a switching state, with a respective open, not yet flooded valve particularly preferably being selected as the starting point, with all the connections determined further preferably being entered in a connection matrix, and with the resulting connection matrix particularly preferably being compared with a control matrix in which all non-permitted connections are entered.

16. A method in accordance with one of the claims 14 or 15, wherein on the recognition of a non-permitted connection on a then current switching state of the valves, the system switches into a safe state and/or on the recognition of a non-permitted connection on a scheduled switching state, this switching state is not brought about.

17. A machine, in particular a medical treatment machine, having a plurality of valve actuators for the control of valves for the flow path control, in particular in a cassette system, having a valve actuation control and having a monitoring unit for the monitoring of the then current and/or scheduled state of a plurality of valves
**characterized in that**
the monitoring unit determines the then current and/or scheduled switching state of the valves and the connections resulting from the switching state of the valves and compares the resulting connections with a predetermined number of non-permitted connections.

18. A machine in accordance with claim 17, wherein the determination of the connections resulting from a switching state always takes place when the switching state of a valve changes or should change, with preferably all the open valves being virtually flooded, starting from one or more starting points, for the determination of the connections resulting from a switching state, with a respective open not yet flooded valve further preferably being selected as the starting point, with all the connections determined selectively being entered in a connection matrix and with the resulting connection matrix preferably being compared with a control matrix in which all the non-permitted connections are entered.

19. A machine in accordance with one of the claims 17 or 18, wherein the machine switches into a safe state on recognition of a non-permitted connection on a then current switching state of the valves and/or does not bring about this switching state on recognition of a non-permitted connection on a scheduled switching state.

20. A machine in accordance with one of the preceding claims 17 to 19 having a coupling surface for the coupling of a cassette which comprises valves and liquid conducting passages, wherein the valve actuators on the machine side determine the switching state of the valves on the cassette side to establish different flow paths in the cassette.

21. A computer program product, in particular a storage medium, having a computer program, for transfer to a machine, in particular a medical treatment machine, having commands for the carrying out of a method in accordance with one of the claims 1 to 6 and 14 to 16.

## Revendications

1. Procédé d'actionnement de valves pour la commande de voies d'écoulement, en particulier dans une machine de traitement médical, comportant une pluralité de processus pour la réalisation des voies d'écoulement par l'intermédiaire de l'actionnement d'un groupe de valves,
**caractérisé en ce que**
chaque processus sollicite pour lui un groupe de valves approprié à sa mise en oeuvre, de telle sorte que d'autres processus ne peuvent pas modifier la position de commande de ces valves, et **en ce que** chaque processus décide lui-même du déblocage des valves sollicitées par lui.

2. Procédé selon la revendication 1, dans lequel chaque processus décide selon des critères internes et/ou externes du déblocage des valves sollicitées par lui, sachant qu'en particulier chaque processus débloque, lorsqu'il est terminé, le groupe de valves sollicité.

3. Procédé selon l'une quelconque des revendications précédentes, dans lequel un processus peut solliciter différents groupes de valves en fonction de la disponibilité des valves, chaque processus interrogeant au choix, avant sa mise en oeuvre, la disponibilité d'un groupe de valves approprié à sa mise en oeuvre, et dans lequel un processus est à nouveau mis en oeuvre au choix lorsqu'un groupe de valves approprié est disponible, le processus sollicitant pour lui ledit groupe de valves.

4. Procédé selon la revendication 3, dans lequel l'interrogation d'une valve non disponible par un deuxième processus conduit à demander au premier processus, qui a sollicité ladite valve, de débloquer ladite valve, sachant que seul le premier processus décide de cette demande, dans lequel procédé le processus qui interroge sollicite pour lui par cette interrogation toutes les valves interrogées et disponibles, et dans lequel, de manière particulièrement préférée, le processus qui interroge obtient par l'interrogation un droit de sollicitation pour toutes les valves interrogées mais non disponibles, de telle sorte qu'il peut solliciter pour lui lesdites valves dès qu'elles sont débloquées.

5. Procédé selon l'une quelconque des revendications précédentes, comportant un processus central d'association des valves, dans lequel tous les autres processus sollicitent, débloquent et/ou interrogent les valves nécessaires.

6. Procédé selon l'une quelconque des revendications précédentes, comportant les étapes:
- solliciter un premier groupe de valves pour la mise en oeuvre d'un premier processus, dans lequel une première voie d'écoulement est établie par l'actionnement du premier groupe de valves associé;
- interroger la disponibilité d'un deuxième groupe de valves pour la mise en oeuvre d'un deuxième processus, dans lequel une deuxième voie d'écoulement est destinée à être établie par l'actionnement du deuxième groupe de valves associé;
- mettre en oeuvre le deuxième processus, lorsque toutes les vannes nécessaires sont disponibles;
sachant que l'interrogation des valves non disponibles par le deuxième processus conduit à demander au premier processus de débloquer lesdites valves, seul le premier processus décidant de cette demande.

7. Machine, en particulier machine de traitement médical, comportant une pluralité d'actionneurs de valves destinés à actionner des valves pour la commande de voies d'écoulement, en particulier dans un système à cassettes, comportant une commande d'actionneurs de valves pour la mise en oeuvre d'une pluralité de processus destinés à établir des voies d'écoulement par l'actionnement d'un groupe d'actionneurs de valves,
**caractérisée en ce que**
la commande d'actionneurs de valves est configurée de telle sorte que chaque processus sollicite pour lui un groupe d'actionneurs de valves approprié à sa mise en oeuvre, de telle sorte que d'autres processus ne peuvent pas modifier la position de commande de ces actionneurs de valves, et chaque processus décide lui-même du déblocage des actionneurs de valves sollicités par lui.

8. Machine selon la revendication 7, dans laquelle chaque processus décide selon des critères internes et/ou externes du déblocage des actionneurs de valves sollicités par lui, sachant que, de préférence, chaque processus débloque, lorsqu'il est terminé, le groupe d' actionneurs de valves sollicité, dans laquelle machine, de préférence, un processus peut solliciter différents groupes d'actionneurs de valves en fonction de la disponibilité des actionneurs de valves, et dans laquelle, de manière particulièrement préférée, chaque processus, avant sa mise en oeuvre, interroge la disponibilité d'un groupe d'actionneurs de valves approprié à sa mise en oeuvre, et dans laquelle un processus est mis en oeuvre au choix lorsqu'un groupe approprié d'actionneurs de valves est disponible, le processus sollicitant pour lui ce groupe d'actionneurs de valves.

9. Machine selon la revendication 8, dans laquelle l'interrogation d'un actionneur de valve non disponible par un deuxième processus conduit à demander au premier processus, qui a sollicité ledit actionneur de valve, de débloquer ledit actionneur de valve, sachant que seul le premier processus décide de cette demande, le processus qui interroge sollicitant pour lui par cette interrogation, de préférence, tous les actionneurs de valves interrogés et disponibles.

10. Machine selon la revendication 9, dans laquelle le processus qui interroge, obtient par l'interrogation un droit de sollicitation pour tous les actionneurs de valves interrogés mais non disponibles, de telle sorte qu'il peut solliciter pour lui lesdits actionneurs de valves dès qu'ils sont débloqués.

11. Machine selon l'une quelconque des revendications 7 à 10, dans laquelle la commande d'actionneurs de valves comprend un processus central d'association des valves, dans lequel tous les autres processus sollicitent, débloquent et/ou interrogent les actionneurs de valves nécessaires.

12. Machine selon l'une quelconque des revendications 7 à 11, dans laquelle les étapes suivantes sont mises en oeuvre:
- solliciter un premier groupe d'actionneurs de valves pour la mise en oeuvre d'un premier processus, dans lequel une première voie d'écoulement est établie par l'actionnement du premier groupe de valves associé;
- interroger la disponibilité d'un deuxième groupe d'actionneurs de valves pour la mise en oeuvre d'un deuxième processus, dans lequel une deuxième voie d'écoulement est destinée à être établie par l'actionnement du deuxième groupe de valves associé;
- mettre en oeuvre le deuxième processus, lorsque tous les actionneurs de valves nécessaires sont disponibles;
sachant que l'interrogation des actionneurs de valves non disponibles par le deuxième processus conduit à demander au premier processus de débloquer lesdits actionneurs de valves, seul le premier processus décidant de cette demande.

13. Machine selon l'une quelconque des revendications 7 à 12, comportant une surface de couplage pour coupler une cassette qui contient les valves et les conduits parcourus par le liquide, dans laquelle les actionneurs de valves du côté de la machine, destinés à établir différentes voies d'écoulement dans la cassette, déterminent la position de commande des valves du côté de la cassette.

14. Procédé de contrôle de l'état actuel et/ou programmé d'une pluralité de valves pendant la commande des voies d'écoulement, en particulier dans une machine de traitement médical,
**caractérisé en ce que**
le procédé comporte les étapes suivantes:
- déterminer la position de commande actuelle et/ou programmée des valves,
- déterminer les liaisons résultant de la position de commande des valves,
- comparer les liaisons résultantes à une quantité prédéfinie de liaisons interdites.

15. Procédé selon la revendication 14, dans lequel les liaisons résultant d'une position de commande sont toujours déterminées lorsque la position de commande d'une valve change ou doit changer, sachant que pour déterminer les liaisons résultant d'une position de commande, de préférence toutes les valves ouvertes sont parcourues virtuellement par un flux à partir d'un ou de plusieurs points de départ, dans lequel, de manière particulièrement préférée, le point de départ choisi est dans chaque cas une valve ouverte, non encore parcourue par un flux, dans lequel, encore mieux, toutes les liaisons déterminées sont enregistrées dans une matrice des liaisons, et dans lequel, de manière particulièrement préférée, la matrice des liaisons qui en résulte est comparée à une matrice de contrôle dans laquelle sont enregistrées toutes les liaisons interdites.

16. Procédé selon la revendication 14 ou 15, dans lequel en cas de détection d'une liaison interdite en présence d'une position de commande actuelle des valves, le système commute dans une position sûre, et/ou en cas de détection d'une liaison interdite en présence d'une position de commande programmée, cette position de commande ne sera pas établie.

17. Machine, en particulier machine de traitement médical, comportant une pluralité d'actionneurs de valves destinés à actionner des valves pour la commande des voies d'écoulement, en particulier dans un système à cassettes, comportant une commande d'actionneurs de valves et comportant une unité de contrôle destinée à contrôler l'état actuel et/ou programmé d'une pluralité de valves,
**caractérisée en ce que**
l'unité de contrôle détermine la position de commande actuelle et/ou programmée des valves et les liaisons résultant de la position de commande des valves, et compare les liaisons résultantes à une quantité prédéfinie de liaisons interdites.

18. Machine selon la revendication 17, dans laquelle les liaisons résultant d'une position de commande sont toujours déterminées lorsque la position de commande d'une valve change ou doit changer, sachant que, de préférence, pour déterminer les liaisons résultant d'une position de commande toutes les valves ouvertes sont parcourues virtuellement par un flux à partir d'un ou de plusieurs points de départ, dans laquelle, encore mieux, le point de départ choisi est de préférence dans chaque cas une valve ouverte, non encore parcourue par un flux, dans laquelle, au choix, toutes les liaisons déterminées sont enregistrées dans une matrice des liaisons, et dans laquelle la matrice des liaisons qui en résulte est comparée de préférence à une matrice de contrôle dans laquelle sont enregistrées toutes les liaisons interdites.

19. Machine selon la revendication 17 ou 18, dans laquelle, en cas de détection d'une liaison interdite en présence d'une position de commande actuelle des valves, la machine commute dans une position sûre, et/ou en cas de détection d'une liaison interdite en présence d'une position de commande programmée, cette position de commande ne sera pas établie.

20. Machine selon l'une quelconque des revendications 17 à 19, comportant une surface de couplage pour coupler une cassette qui contient les valves et les conduits parcourus par le liquide, dans laquelle les actionneurs de valves du côté de la machine, destinés à établir différentes voies d'écoulement dans la cassette, déterminent la position de commande des valves du côté de la cassette.

21. Produit de programme d'ordinateur, en particulier support de mémoire avec un programme d'ordinateur à faire dérouler sur une machine, en particulier une machine de traitement médical, avec des instructions pour la mise en oeuvre d'un procédé selon l'une des revendications 1 à 6 et 14 à 16.
